# EUROPEAN PATENT APPLICATION

(11) **EP 4 066 784 A1**
(43) Date of publication of application: **05.10.2022**
(21) Application number: 20893449.7
(22) Date of filing: 13.11.2020
(51) Int. Cl.: A61F 2/01, A61B 90/00

(54) **EMBOLIC PROTECTION DEVICE**

(30) Priority: 28.11.2019 CN 201911193147; 25.12.2019 CN 201911360871; 25.12.2019 CN 201911360876; 25.12.2019 CN 201911359116; 25.12.2019 CN 201911359113
(71) Applicant: Shanghai Microport Shield Medtech Co., Ltd., China (Shanghai) Pilot Free Trade Zone Shanghai 201203 (CN)
(72) Inventor: NI, Zunzhang, Shanghai 201203 (CN); SUN, Jianfeng, Shanghai 201203 (CN); ZHANG, Ziheng, Shanghai 201203 (CN); LI, Shuang, Shanghai 201203 (CN); JIN, Qiaorong, Shanghai 201203 (CN); LUO, Qiyi, Shanghai 201203 (CN)
(74) Representative: Lavoix
(86) International application number: PCT/CN2020/128863
(87) International publication number: WO 2021/104063

(57) **Abstract**

An embolic protection device includes an embolic diverter (100) and a push tube (300). The embolic diverter (100) includes a first support frame (110) and a first filter member (120) provided on the first support frame (110). The push tube (300) includes a tubular body (310) coupled to the embolic diverter (100) and adapted to advance, position and adjust the embolic diverter (100). The push tube (300) enables the embolic diverter (100) that is intended to protect upstream blood vessels to have excellent wall conformity. The tubular body (310) further includes a distal cut hypotube section, which allows the push tube (300) to be shaped by a guidewire disposed therein and thus self-adapt to the anatomy of a patient's aortic arch and additionally enhance the wall conformity of the embolic diverter (100).

## Description

### TECHNICAL FIELD

The present application relates to the field of medical devices and, in particular, to embolic protection devices for preventing the occurrence of embolism during surgery.

### BACKGROUND

During cardiac or aortic procedures, such as cardiac surgical procedures, cardiopulmonary bypass procedures, catheter-based interventional cardiac procedures and ascending aortic surgical procedures, clotting materials may be produced, such as blood clots, fat droplets, bacterial clumps, platelet aggregates induced by tumor or other cells, or atherosclerotic fragments dislodged from the arterial wall. When transported by the blood stream into the brain, these materials may occlude a small artery somewhere in the brain and lead to an embolism there. Cerebral embolisms have been recognized as an important complication of cardiac and aortic surgery.

For example, out of every 10 patients who have undergone transcatheter aortic valve replacement (TAVR) procedures, one developed a clinically significant stroke attributed to the procedure. A majority of such strokes occurred during or within 72 hours after surgery. Analysis revealed that they might be attributed to cerebral embolisms caused by debris that were dislodged from the native heart valves or from the aortic wall and migrated to the brain.

In order to prevent complications caused by clotting materials, embolic protection devices are often used in surgical procedures, which can benefit patients by diverting or collecting plaques, debris, blood clots or other clotting materials that are directed toward the brain before they can enter the brain and possibly cause an embolism there. However, existing embolic protection devices are associated with a number of problems including operating complexity, poor wall conformity and escape of emboli.

### SUMMARY OF THE INVENTION

It is an aim of the present application to overcome poor overall wall conformity and other problems with conventional embolic protection devices by presenting novel embolic protection devices.

In pursuit of this aim, the present application provides an embolic protection device including:
an embolic diverter including a first support frame and a first filter member disposed on the first support frame; and
a push tube including a tubular body coupled to the embolic diverter, the tubular body adapted to advance, position and adjust the embolic diverter.

Optionally, the push tube may further include a first coupling element provided on the tubular body, wherein the tubular body is coupled to the embolic diverter by the first coupling element.

Optionally, the first coupling element may define a through hole, through which the first support frame or the first filter member is proximally passed, thereby coupling the embolic diverter to the push tube.

Additionally, the through hole may extend through the tubular body in a direction perpendicular to an axis of the tubular body.

Additionally, the first coupling element may include a first tubular element, a first connecting bar and a second tubular element, which are sequentially arranged along a direction from a distal end of the tubular body to a proximal end thereof, the first and second tubular elements both disposed coaxially with and over the tubular body and coupled to each other by the first connecting bar.

Further, the first connecting bar may be elongate and convex away from the tubular body, thus forming the through hole between the first connecting bar and the tubular body.

Further, the first coupling element may include a third tubular element, a second connecting bar and a fourth tubular element, which are sequentially arranged along a direction from the distal end of the tubular body to the proximal end thereof, the fourth tubular element disposed coaxially with and over the tubular body and coupled to the third tubular element by the second connecting bar, the third tubular element having an axis that is perpendicular to the axis of the tubular body, the third tubular element defining a lumen which provides the through hole.

Optionally, the tubular body may further include a distal cut hypotube section having a length between 80 mm and 350 mm along the axis of the tubular body.

Optionally, the cut hypotube section may include a plurality of cut units extending helically along the axis of the tubular body.

Optionally, the cut hypotube section may include a plurality of cut portions spaced apart along the axis of the tubular body, each including at least one cut unit spaced apart circumferentially around the tubular body.

Optionally, the cut hypotube section may include a plurality of cut portions spaced apart circumferentially around the tubular body, each including at least one cut unit spaced apart along the axis of the tubular body.

Additionally, each cut unit may include at least one groove.

Additionally, the groove may include at least one circular end recess and an elongate groove connecting the circular recess.

Additionally, the elongate groove may include at least one V-shaped structure and be brought into communication with the circular recess.

Additionally, the direction of extension of each cut unit may form an angle ranging from 60 degrees to 90 degrees with the axis of the tubular body.

Additionally, a ratio of a length of each cut unit extending on an outer surface of the tubular body to a circumferential length of the outer surface of the tubular body may range from 0.08 to 0.90.

Optionally, the embolic protection device may further include an embolic collector including a second support frame and a second filter member attached to the second support frame, wherein the push tube further includes a second coupling element disposed on the tubular body between the first coupling element and the proximal end of the tubular body, and wherein the embolic collector is coupled to the push tube by the second coupling element.

Additionally, the second coupling element may include a groove running circumferentially around the tubular body, wherein the embolic collector is disposed at the groove and thus coupled to the push tube.

Optionally, the embolic protection device may further include an embolic collector including: a proximal connection; a second support frame including a plurality of support struts spaced apart about an axis, at least some of which are curved to define a concave surface facing the axis, each of the support struts having a first proximal end and an opposing first distal end along a direction parallel to the axis, the first proximal ends of the support struts gathered at the proximal connection; and a second filter member having a second proximal end and an opposing second distal end, the second filter member provided on the second support frame, the second proximal end provided at the first proximal ends of the support struts, the second distal end provided on the second support frame to form an opening.

Optionally, the push tube may be a straight structure having an outer diameter kept constant from a proximal end of the push tube to a distal end thereof. Alternatively, the push tube may be a tapered structure that it is wide at the proximal end and narrow at the distal end.

Optionally, the embolic diverter may further include at least one first support strut attached to the first support frame at joints scattered on opposite sides of a longitudinal centerline of the first support frame.

Optionally, the first filter member may have at least two concave surfaces all facing a plane defined by the support frame.

Optionally, the embolic protection device may further include a delivery sheath including an inner layer, and both disposed externally around the inner layer, a helical layer and a braided layer, the braided layer being a meshed structure braided from interlaced first filaments, the helical layer being a helical structure formed by axially coiled second filaments, the delivery sheath including at least two axially-extending segments, in at least one of which, an angle formed by the first and second filaments differs from an angle formed by the first and second filaments in another one of the segments.

The present application provides another embolic protection device, including:
a push tube having a first proximal end and an opposing first distal end;
an embolic diverter provided on the push tube, the embolic diverter including a first filter member; and
an embolic collector coaxially disposed on the push tube between the embolic diverter and the first proximal end, the embolic collector including a second support member and a second filter member provided on the second support member, the second filter member having a second proximal end and an opposing second distal end, the second filter member being closed at the first proximal end and defining an opening at the second distal end, the opening adapted to allow clots to enter and thus be trapped in the second filter member.

The present application provides the following advantages over the prior art:
It provides an embolic protection device including: an embolic diverter and a push tube, the embolic diverter including a first support frame and a first filter member provided on the first support frame; and a push tube including a tubular body coupled to the embolic diverter, the push tube adapted to advance, position and adjust the embolic diverter, the tubular body having a distal cut hypotube section. According to the present application, the push tube enables the embolic diverter that is intended to protect upstream blood vessels to have excellent wall conformity. The distal cut hypotube section of the tubular body allows the tubular body to be shaped by a guidewire disposed therein and thus self-adapt to the anatomy of a patient's aortic arch and additionally enhance the embolic diverter's wall conformity. The push tube further includes a first coupling element defining a through hole, through which the first support frame or the first filter member is proximally passed, thus coupling the embolic diverter to the push tube. The first coupling element enables the push tube to deliver better pushing and manipulating forces, imparting desirable degrees of freedom to the embolic protection device. The embolic protection device is simple in design, comprehensive in functionality and convenient to manipulate.

### BRIEF DESCRIPTION OF THE DRAWINGS

Fig. 1 is a schematic illustration of blood vessels arising from the aortic arch;
Fig. 2 is a schematic diagram showing the structure of an embolic protection device, according to an embodiment of the present application;
Fig. 3 schematically illustrates the embolic protection device that is being deployed in the aortic arch of Fig. 1, according to an embodiment of the present application;
Fig. 4 is a schematic diagram showing the structure of an embolic diverter, according to an embodiment of the present application;
Figs. 5a to 5b are schematic diagrams showing the structure of a support body, according to an embodiment of the present application;
Figs. 6a to 6b are schematic diagrams showing the structure of another support body, according to an embodiment of the present application;
Fig. 7 is a schematic diagram showing the structure of the embolic diverter, according to an embodiment of the present application;
Fig. 8 is a projection of the embolic diverter of Fig. 7 on an X-Y plane;
Fig. 9 is a schematic diagram showing the structure of a support frame in the embolic diverter, according to an embodiment of the present application, which is formed by splicing one pre-curved wire;
Fig. 10 is a schematic diagram showing the structure of a variant of the support frame in the embolic diverter, according to an embodiment of the present application, which is formed by splicing two pre-curved wires;
Fig. 11a is a schematic illustration of a first proximal anti-flex feature in the embolic diverter, according to an embodiment of the present application, which is V-shaped;
Fig. 11b is a schematic illustration of a variant of the first proximal anti-flex feature in the embolic diverter, according to an embodiment of the present application, which is a curved structure;
Fig. 12a is a schematic diagram showing the structure of another variant of the support frame in the embolic diverter of Fig. 1;
Fig. 12b is a schematic diagram showing the structure of a further variant of the support frame in the embolic diverter of Fig. 1;
Fig. 13 is a schematic diagram showing the structure of a support frame in the embolic diverter, according to an embodiment of the present application, which is a curved three-dimensional structure;
Fig. 14 is a schematic diagram showing the structure of a support frame in the embolic diverter, according to an embodiment of the present application, in which the middle part of the support frame tapers inward;
Figs. 15a to 15c are schematic diagrams showing the structure of a second support frame, according to an embodiment of the present application;
Fig. 16a is a schematic diagram showing the structure of a first embodiment of an embolic collector, according to the present application;
Fig. 16b is a cross-sectional view of the embolic collector of Fig. 16a along A-A, not showing a proximal connection and showing support struts only by their cross-sections;
Fig. 17 is a schematic illustration of a variant of the embolic collector of Fig. 16a, not showing a filter member which is intended to be not fitted at middle points of support struts;
Fig. 18 is a schematic illustration of another variant of the embolic collector of Fig. 16a, not showing the filter member and showing the support struts which are unequidistantly arranged circumferentially with respect to the embolic collector;
Fig. 19a is a schematic diagram showing the structure of a second embodiment of the embolic collector, according to the present application, not showing the filter member;
Fig. 19b is a cross-sectional view of the embolic collector of Fig. 19a;
Fig. 20 is a schematic diagram showing the structure of a third embodiment of the embolic collector, according to the present application, not showing the filter member;
Figs. 21a to 21e are schematic diagrams showing possible structures of a tubular body, according to embodiments of the present application;
Figs. 22a to 22e are schematic diagrams showing the structures of variants of a coupling element, according to embodiments of the present application;
Fig. 23 is a schematic perspective view of a delivery sheath, according to an embodiment of the present application;
Fig. 24 is an axial cross-sectional view of the delivery sheath, according to an embodiment of the present application;
Fig. 25 is a schematic diagram showing the structure of a helical layer, according to an embodiment of the present application;
Fig. 26 is a schematic diagram showing the structure of the helical layer and a braided layer forming an angle with the helical layer, according to an embodiment of the present application;
Fig. 27 is a schematic diagram showing the structure of an embolic protection system, according to an embodiment of the present application; and
Fig. 28 schematically illustrates how an embolus diverter and an embolus capture device are coupled to a push tube in the embolic protection system, in accordance with an embodiment of the present application.

### Reference Numerals in the Drawings:

11 - Ascending Aorta; 12 - Descending Aorta; 13 - Aortic Arch;
21 - Brachiocephalic Trunk Artery; 22 - Left Common Carotid Artery; 23 - Left Subclavian Artery;
100 - Embolic Diverter; 110 - First Support Frame; 111 - Support Body; 112 - First Support Strut; 112a - First Support Point; 113 - Second Support Strut; 113a - Second Support Point; 114 - Second Opening; 115 - First Distal Anti-flex Feature; 116 - Splice Joint; 117 - First Opening; 118 - First Proximal Anti-flex Feature; 120 - First Filter Member;
200 - Embolic Collector; 210 - Second Support Frame; 220 - Second Filter Member; 230 - Proximal Connection; 121 - Support Strut, 121a - Third Support Strut, 121b - Fourth Support Strut, 121c - Fifth Support Strut, 121d - First Support Strut, 121e - Second Support Strut, 122 - Recessed Portion; 240 - Distal Connection;
300 - Push Tube; 310 - Tubular Body; 310a - Cut Portion; 311 - Cut unit; 320 - First Coupling Element; 321 - Through Hole; 322 - First Tubular Element; 323 - Second Tubular Element; 324 - First Connecting Bar; 325 - Third Tubular Element; 326 - Fourth Tubular Element; 327 - Second Connecting Bar;
400 - Delivery Sheath; 401 - Outer Layer; 402 - Helical Layer; 403 - Braided Layer; 404 - Inner Layer;
500 - Guide Member;
700 - Handle.

### DETAILED DESCRIPTION

Embolic protection devices according to the present application will be described in greater detail below with reference to the accompanying schematic drawings, which present preferred embodiments of the invention. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of an actual implementation are described in this specification. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

Objects, features and advantages of the present application will become more apparent upon reading the following more detailed description thereof with reference to the accompanying drawings. Note that the figures are provided in a very simplified form not necessarily drawn to exact scale for the only purpose of facilitating easy and clear despription of the disclosed embodiments.

As used herein, the terms "proximal" and "distal" are employed to describe relative orientations, relative positions and directions between components of a medical device or actions thereof, as viewed by a physician operating the device. Without wishing to be limiting, a "distal end" usually refers to an end that enters a patient first, and a "proximal end" usually refers to an end closer to the operator, during normal operation of the medical device. The term "or" is generally employed in the sense of "and/or", unless the context clearly dictates otherwise. As used herein, the terms "inner", "outer" and similar terms are merely illustrative and do not represent the only implementation possible.

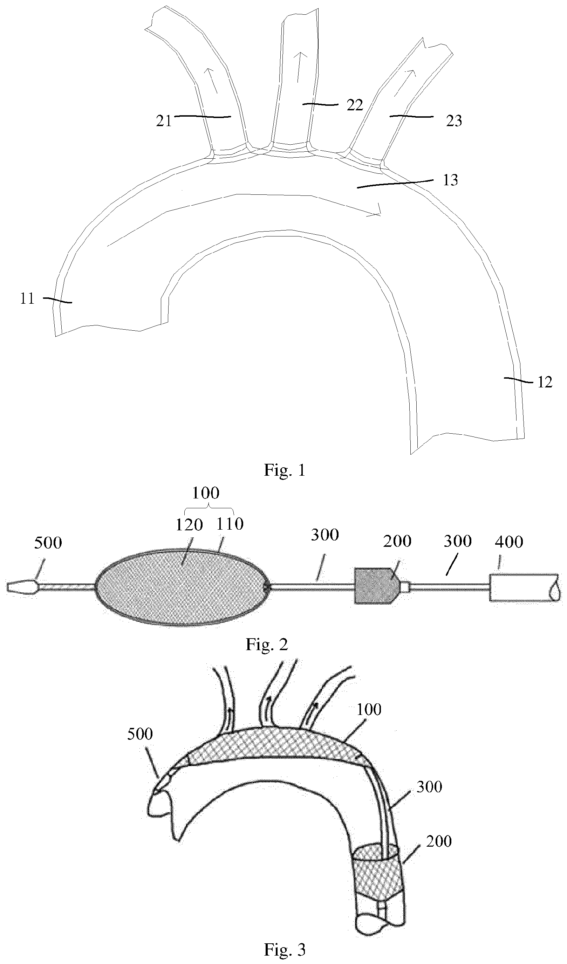

Fig. 1 is a schematic illustration of blood vessels arising from the aortic arch. As shown in Fig. 1, before a transcatheter procedure in which clotting materials may be produced begins, an embolic protection device according to an embodiment of the present application may be surgically deployed in the ascending aorta 11, the aortic arch 13 and/or the descending aorta 12 to filter blood ascending toward the branch blood vessels, i.e., the brachiocephalic trunk artery 21, the left common carotid artery 22 and the left subclavian artery 23, and blood flowing toward downstream blood vessels to prevent clotting materials that may be produced in the surgical procedure from entering the brain or the downstream blood vessels and thus possibly causing a stroke in the brain or a complication in a downstream organ, such as acute kidney injury. The embolic protection device may be introduced, for example, from the femoral artery, which can result in a shorter delivery path and hence reduced surgical complexity.

Fig. 2 is a schematic diagram showing the structure of the embolic protection device of the present embodiment. As shown in Fig. 2, the embolic protection device may include an embolic diverter 100, an embolic collector 200, a push tube 300 and a delivery sheath 400. The embolic diverter 100 is coupled to the embolic collector 200 and the delivery sheath 400 by the push tube 300. The delivery sheath 400 is connected on the side of the embolic collector 200 distal from the embolic diverter 100 and adapted to accommodate the embolic diverter 100 and the embolic collector 200, both in a crimped configuration, as well as the push tube 300. The push tube 300 is adapted to advance, position and adjust the embolic diverter 100 and the embolic collector 200. Specifically, a proximal end of the push tube 300 is configured to enable release or retrieval of the embolic diverter 100 and the embolic collector 200.

Fig. 3 schematically illustrates the embolic protection device of the present embodiment that is being deployed in the aortic arch of Fig. 1. As shown in Fig. 3, the embolic protection device is located between the proximal end of the ascending aorta and the distal end of the descending aorta and fits against the wall of the aortic arch. Specifically, the embolic diverter 100 is positioned within the aortic arch in order to filter blood ascending toward the brachiocephalic trunk artery 21, the left common carotid artery 22 and the left subclavian artery 23 to prevent blood clots that may be produced in the surgical procedure from entering the brain and thus possibly causing a stroke. The embolic collector 200 is located between the aortic arch and the distal end of the descending aorta 12 to filter blood flowing toward downstream blood vessels to prevent clotting materials that may be produced in the surgical procedure from entering a downstream organ and thus possibly causing a complication such as acute kidney injury.

With continued reference to Fig. 2, the embolic diverter 100 may include a first support frame 110 and a first filter member 120. The push tube 300 is shaped by a guidewire to support the first support frame 110 and first filter member 120, thus improving the wall conformity of the embolic diverter 100 and reducing interference of the embolic diverter 100 with any subsequent medical instrument.

The first support frame 110 is adapted to support the first filter member 120 and define a circumferential coverage area thereof so that the entrances of the branch blood vessels arising from the aortic arch are all covered. The first support frame 110 may be braided from filaments, or formed by cutting. The first support frame 110 may be a circumferentially closed annular structure. In the present embodiment, the first support frame 110 may be a three-dimensional structure such as a convex structure. That is, the first support frame 110 defines a curved plane. Such a convex three-dimensional structure allows the first filter member 120 attached to the first support frame 110 to more closely fit against the inner wall of the aortic arch, thus avoiding the escape of clotting materials. In other embodiments, the first support frame 110 may be alternatively a flat structure. That is, the first support frame 110 defines a flat plane. The first support frame 110 may also be a polygonal, elliptic, quasi-elliptic or other structure. The first support frame 110 may be either a symmetrical or asymmetrical structure. In the present embodiment, the first support frame 110 is elliptic. This shape enables the structure to be desirably elastic and easily collapsible.

The first filter member 120 may be a first filter screen, which is attached to the first support frame 110 and adapted to remove blood clots and other clotting materials from blood flowing through the first filter member 120. Specifically, the first filter member 120 may cover the first support frame 110 so as to be able to remove blood clots from blood flowing through the embolic diverter 100. The first filter member 120 may be a three-dimensional structure such as a convex structure. That is, the first filter member 120 defines a curved plane. Such a convex three-dimensional structure allows the first filter member 120 to more closely fit against the inner wall of the aortic arch, thus avoiding the escape of clotting materials. In other embodiments, the first filter member 120 may be alternatively a flat structure. That is, the first filter member 120 defines a flat plane. The first filter member 120 may be attached to the first support frame 110 on either or both sides thereof in such a manner that peripheral edges of the first filter member 120 are tied to the first support frame 110. The first filter member 120 may have a smaller opening size when it is attached to the first support frame 110 on one side thereof than when it is attached to the first support frame 110 on both sides thereof. The opening size of the first filter member 120 may be between 60 µm and 250 µm. A total area of openings in the first filter member 120 may account for 40-70% of a total area of the first filter member 120, which can block the passage of clots therethrough while ensuring that the blood flow rate is not affected. The first filter member 120 may be braided from filaments of a biocompatible material such as a nickel-titanium alloy, a polymer or an inorganic non-metallic material. Alternatively, it may be formed by punching a sheet of the same material. The first filter member 120 may be either elastic or not.

Optionally, the first filter member 120 may be coated with a coating. The coating may be heparin, an anticoagulant, etc. It can avoid occlusion of some openings in the first filter member 120 due to accumulation therein of blood clots that may be produced in the surgical procedure. Alternatively, the first filter member 120 may be fabricated from an anticoagulant material with anti-clotting or similar properties.

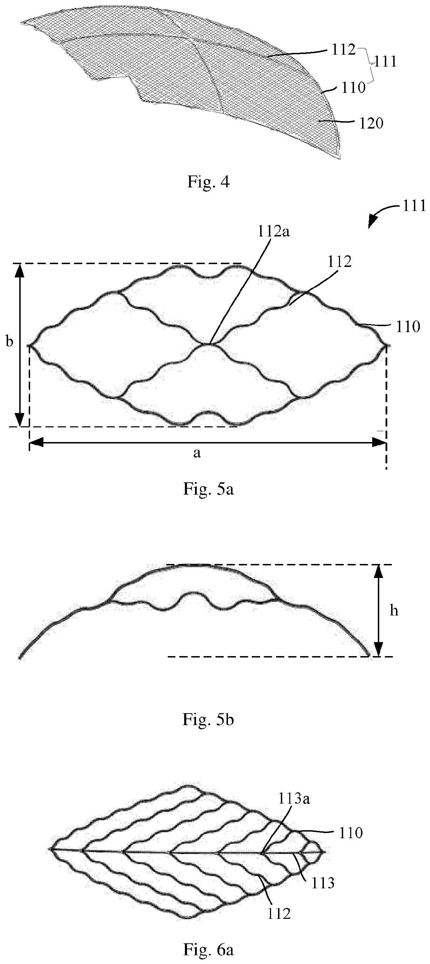

Fig. 4 is a schematic diagram showing the structure of an embolic diverter 100 according to another embodiment of the present application. As shown in Fig. 4, the embolic diverter 100 may include a support body 111 and a first filter member 120. The support body 111 is adapted to support the first filter member 120 and define a circumferential coverage area of the first filter member 120 so that the entrances of the branch blood vessels arising from the aortic arch are all covered. The support body 111 may include a first support frame 110 and at least one first support strut 112. The first support strut 112 is attached to the first support frame 110 at joints scattered on opposite sides of a longitudinal centerline of the first support frame 110.

Figs. 5a to 5b are schematic diagrams showing the structure of the support body according to an embodiment of the present application. As shown in Figs. 5a and 5b, in the present embodiment, the first support frame 110 is a parallelogram. This shape enables the structure to be desirably elastic and easily collapsible. In other embodiments, the first support frame 110 may be alternatively pentagonal, hexagonal or otherwise shaped. The sides of the first support frame 110 may be equally long or not. A longitudinal length of the first support frame 110 may be not greater than 120 mm, in particular, 50 mm, 60 mm, 70 mm, 80 mm, 90 mm, 100 mm, 110 mm, 120 mm, etc. A transverse width of the first support frame 110 may be not greater than 100 mm, in particular, 50 mm, 60 mm, 70 mm, 80 mm, 90 mm or 100 mm, etc. The first support frame 110 may define a curved plane with a height difference between the highest and lowest points that is between 5 mm and 30 mm, in particular 5 mm, 10 mm, 15 mm, 20 mm, 25 mm, 30 mm, etc.

As shown in Fig. 5a, the support body 111 includes the at least one first support strut 112, the first support strut 112 is attached to the first support frame 110. The first support strut 112 may extend either in or above the plane defined by the first support frame 110. The first support strut 112 is adapted to support the first filter member 120 to prevent the first filter member 120 from being depressed or corrugated and thus possibly blocking any subsequent instrument. The first support strut 112 may be integrally formed with the first support frame 110, or the first support strut 112 may be fixed to the first support frame 110.

In some embodiments, the first support strut 112 may be curved so as to be concave toward the plane defined by the first support frame 110. Supported by the curved first support strut 112, the first filter member 120 will bulge toward the branch blood vessels, thus more closely fitting against the vascular wall, and leave a greater space for the passage of subsequent instruments.

In some embodiments, the support body 111 may include at least two first support struts 112 and at least one first support point 112a. The first support struts 112 are connected to the first support frame 110 and the first support point 112a. The first support frame 110 includes a distal end and a proximal end. The support body 111 may include only one first support point 112a, which may be arranged around the proximal end or the distal end, or the first support point 112a may be arranged between the proximal end and the distal end. The support body 111 may also include at least two first support points 112a that are spaced apart from each other either evenly or not between the distal and proximal ends of the first support frame 110. The present application is not limited to any particular position of each first support point 112a.

In some embodiments, at least two first support struts 112 are connected to a single first support point 112a so that they radiate out from the first support point 112a. In the embodiment shown in Fig. 5a, the support body 111 includes one first support point 112a and four first support struts 112 each attached at one end to the first support point 112a and at the other end to the first support frame 110. Moreover, the joints of two of the first support struts 112 with the first support frame 110 are located on one side of the longitudinal centerline of the first support frame 110, and the joints of the remaining two of the first support struts 112 with the first support frame 110 are located on the other side of the longitudinal centerline of the first support frame 110.

In some embodiments, the first support frame 110 and/or the first support struts 112 may each have a wavy shape with multiple crests and multiple troughs. The multiple crests or the multiple troughs may be evenly distributed along the first support frame 110 or the first support strut 112, and each crest or trough may be of the same amplitude or not. The wavy shape of the first support frame 110 and/or the first support struts 112 enables the support body 111 to be easily collapsed and placed into the sheath. Moreover, it allows the support body 111 to easily adapt to a varying radius of curvature at the aortic arch and increases the compliance and wall conformity of the support body 111. The first support frame 110 and/or the first support struts 112 may be flat and have a maximum cross-sectional length which may be between 0.2 mm and 3 mm, in particular 0.2 mm, 0.4 mm, 0.6 mm, 0.8 mm, 1 mm, 1.2 mm, 1.5 mm, 1.8 mm, 2.0 mm, 2.5 mm, 3 mm, etc.

The first support frame 110 and/or the first support struts 112 may be formed of a biocompatible material such as a metal, a polymer or an inorganic non-metallic material by laser engraving. Alternatively, it may be formed by braiding or coiling one or more wires of a biocompatible material such as a metal, stainless steel or an inorganic non-metallic.

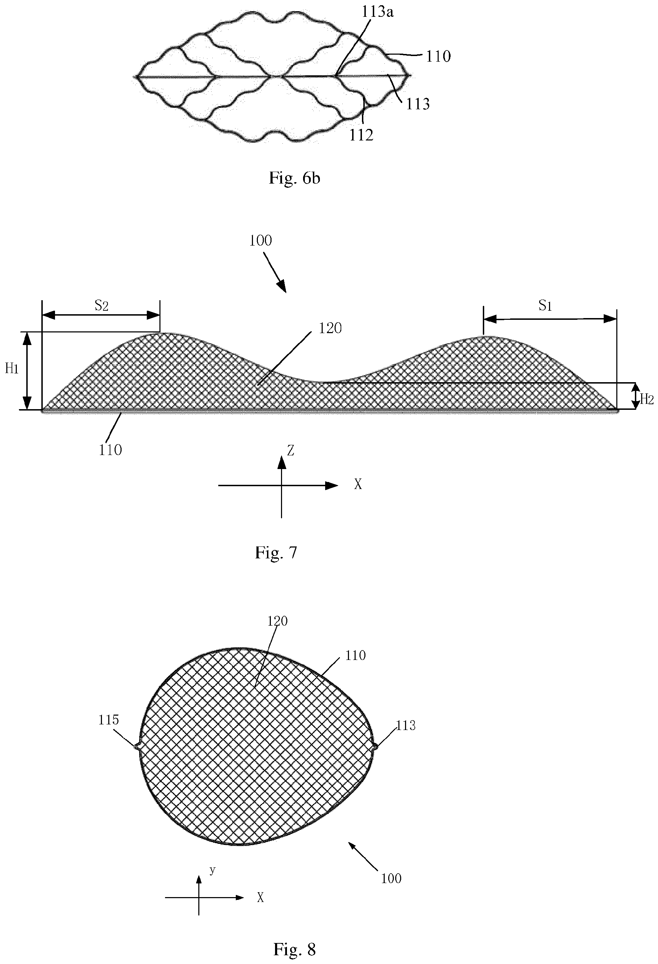

Figs. 6a and 6b are schematic diagrams showing the structure of a variant of the support body according to an embodiment of the present application. As shown in Figs. 6a and 6b, the support body 111 may further include a second support strut 113 connecting the distal and proximal ends of the first support frame 110. The second support strut 113 may have at least one second support point 113a each connected by a first support strut 112 to the first support frame 110.

The second support strut 113 may be either wavy or linear in shape. Preferably, the second support strut 113 has a wavy shape with multiple crests and multiple troughs. The second support strut 113 may alternatively be curved so as to be concave toward the plane defined by the first support frame 110.

In some embodiments, the support body 111 may include at least two, preferably an even number of, first support struts 112 that connect the second support strut 113 to the first support frame 110. The even number first support struts 112 may form one or more pairs each consisting of two first support struts 112 that are connected at the same second support point 113a and are oriented to resemble the letter "V". The pairs of first support struts 112 may be spaced apart between the proximal and distal ends of the first support frame 110. In some embodiments, the pairs of first support struts 112 are all open toward the same end of the first support frame 110, which may be either the proximal or distal end. In other embodiments, at least one pair of first support struts 112 is open toward the proximal end of the first support frame 110, and each remaining pair of first support struts 112 is open toward the distal end of the first support frame 110. For example, the openings formed by the pair of first support struts 112 closer to the proximal end face the proximal end, and the openings formed by the pair of first support struts 112 closer to the distal end face the distal end.

A distance between any adjacent two pairs of first support struts 112 may be determined as required. For example, the first support struts 112 may be arranged densely in a region proximal to the distal end and sparsely in a region distal from the distal end. Alternatively, a density of the first support struts 112 may gradually decrease along a direction from the distal to proximal end. The second support strut 113 may be flat in shape and have a maximum cross-sectional length which may be less than or equal to that of the first support frame 110. The maximum cross-sectional length of the second support strut 113 may be between 0.2 mm and 3 mm, in particular 0.2 mm, 0.4 mm, 0.6 mm, 0.8 mm, 1 mm, 1.2 mm, 1.5 mm, 1.8 mm, 2.0 mm, 2.5 mm, 3 mm, etc.

In another embodiment, the first filter member 120 is configured as a three-dimensional structure having at least two concave surfaces all facing the plane defined by the first support frame 110. This structure enables the embolic diverter 100 to better adapt to the anatomy of the blood vessel. Specifically, after the embolic diverter 100 is delivered and released into the aortic arch 13, under the effect of transverse compression by the wall of the blood vessel, the first support frame 110 is transversely narrowed and longitudinally elongated. If the first filter member 120 is a flat or domed structure, in the transversely narrowed configuration of the first support frame 110, there will be a significant transverse excess of the first filter member 120, which tends to interfere with the operation of subsequent surgical instruments and be damaged by such surgical instruments during their insertion or withdrawal into or from the blood vessel, leading to a degradation in protection. By contrast, in the above embodiment of the present application, the three-dimensional structure having at least two concave surfaces can better adapt the first filter member 120 to changes in radius of curvature and diameter of the blood vessel and lead to little or even no transverse excess, thus effectively avoiding a surgical instrument from coming into contact with the first filter member 120 and thus possibly encountering greater resistance, being blocked or even causing damage to the first filter member 120.

Optionally, the first filter member 120 may have two, three or even more concave surfaces. This embodiment will be described in the context of the first filter member 120 having only two concave surfaces which are continuous with each other in a first direction as an example. Those skilled in the art can adapt it to applications requiring more concave surfaces on the basis of the following description.

With particular reference to Fig. 7, each of the concave surfaces has an apex, and the apices of the two adjacent concave surfaces are connected by a curved line having a lowest point. The apex of each concave surface is spaced from the plane defined by the first support frame 110 by a distance H₁ ranging from 10 mm to 50 mm, preferably from 20 mm to 30 mm. A distance H₂ from the lowest point to the plane defined by the first support frame 110 is in the range of 0-20 mm. In the present embodiment, the distances from the apices of the respective concave surfaces to the plane defined by the first support frame 110 are equal. However, in other embodiments, the distances from the apices of the respective concave surfaces to the plane defined by the first support frame 110 may be unequal. Hereinafter, the two concave surfaces of the first filter member 120 will be referred to respectively as a first concave surface and a second concave surface. The first concave surface is proximal to the first proximal end of the first support frame 110, and the second concave surface to the first distal end of the first support frame 110. Moreover, a distance S1 from an axis of the first concave surface to the first proximal end may be equal to a distance S2 from an axis of the second concave surface to the first distal end or not. In this embodiment, the distance S2 from the axis of the first concave surface to the first proximal end is 10-30 mm, and/or the distance S1 from the axis of the second concave surface to the first distal end is 10-30 mm.

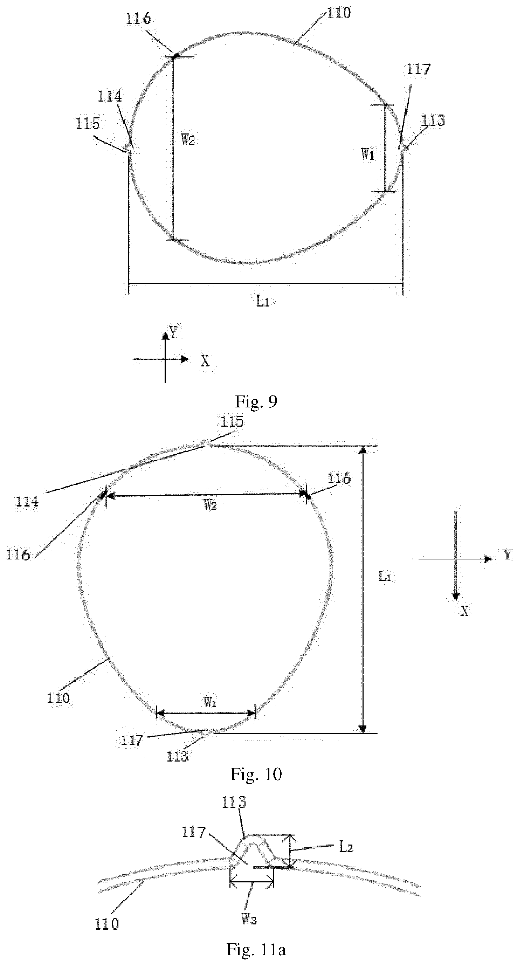

In some embodiments, the first support frame 110 may be formed by cutting a sheet or tube. In some other embodiments, the first support frame 110 may be formed by splicing a pre-curved wire. For example, the first support frame 110 may be formed as a closed annulus by curving a wire and then splicing its opposing ends together by welding or a sleeve. In this case, the first support frame 110 has one splice joint 116, as shown in Fig. 9. Alternatively, the first support frame 110 may be formed as a closed annulus by curving two wires and then splicing their four ends at two splice joints 116, as shown in Fig. 10. The wire(s) spliced to form the first support frame 110 may each consist of either a single strand or twisted multiple strands. A diameter of each wire may range from 0.3 mm to 1 mm, preferably from 0.5 mm to 0.7 mm.

As shown in Fig. 8, in the present embodiment, the first support frame 110 encircles a teardrop-shaped region. In alternative embodiments, the first support frame 110 may encircle a circular, ovate, elliptic or otherwise shaped region. As shown in Figs. 9 and 10, in an exemplary embodiment, the plane defined by the first support frame 110 is a flat plane. The first support frame 110 will be described below as being shaped like a teardrop as an example.

The first support frame 110 has a pre-curved first proximal end section providing the first proximal end and a pre-curved first distal end section providing the first distal end. A radius of curvature of the first proximal end section may be smaller than a radius of curvature of the first distal end section. Alternatively, the first support frame 110 may have a radius of curvature gradually decreasing from the first distal end to the first proximal end. Considering the aorta gradually decreases in diameter from the ascending aorta 11 to the aortic arch 13 to the descending aorta 12, the teardrop-shaped first support frame 110 can well fit the anatomy of the aorta when the embolic diverter 100 is deployed in the aorta with the first distal end being proximal to the ascending aorta 11, the first proximal end being proximal to the descending aorta 12 and the first support frame 110 being arched. This can enhance conformity of the first support frame 110 with the vascular wall and avoid creating any clearance between the first support frame 110 and the inner wall of the blood vessel, through which clots may escape into the three blood vessels.

Depending on the size of the aorta, a length L₁ of the plane defined by the first support frame 110 in the first direction is between 40 mm and 150 mm, and a maximum length of the first support frame 110 in a second direction is between 30 mm and 100 mm. Preferably, the length L₁ of the plane defined by the first support frame 110 in the first direction ranges from 60 mm to 120 mm.

Optionally, as shown in Fig. 10, in a particular embodiment, according to a required curvature radius profile of the first support frame 110, the teardrop-shaped first support frame 110 may be divided into a first arc, first transitions and a second arc, which smoothly transition from one to another. The first arc is located on the first proximal end section, and the second arc on the first distal end section. Thus, a diameter of the first arc is smaller than a diameter of the second arc. A width W₁ of the first arc (i.e., its size in the second direction) may range from 20 mm to 80 mm, and a width W₂ of the second arc (i.e., its size in the second direction) may range from 30 mm to 120 mm. The width W₁ of the first arc may range from 30 mm to 50 mm, and the width W₂ of the second arc may range from 60 mm to 100 mm.

As shown in Fig. 10, in the present embodiment, the first support frame 110 is composed of two pre-curved wires that are joined together. Hereinafter, these wires will be referred to respectively as a first wire and a second wire. The first support frame 110 is divided by a boundary line connecting the joints 111 of the first and second wires into the first proximal end section and the first distal end section. The first wire provides the first distal end section of the first support frame 110, and the second wire provides the first proximal end section of the first support frame 110. Preferably, the first distal end section is shorter than the first proximal end section in the first direction, and the first wire has a greater radial size than the second wire. This structure is advantageous in that, in conformity with the blood vessel's profile with a diameter gradually decreasing from the ascending aorta 11 to the descending aorta 12, the first support frame 110 can be supported in balance when released at the aortic arch 13, and in that a smaller supporting force is needed at the first proximal end than at the first distal end, which allows the embolic diverter 100 to be easily crimped and pre-loaded in a delivery system for delivery and to encounter reduced resistance during delivery.

Additionally, as shown in Figs. 8, 9 and 10, the first support frame 110 defines several anti-flex features. When the embolic diverter 100 is crimped in the delivery device, these anti-flex features can improve anti-flex performance of the first support frame 110 and avoid its breakage under flexture. Moreover, during release of the embolic diverter 100, the anti-flex features can enhance outward expandability of the first support frame 110, facilitating its return to the initial configuration (i.e., the teardrop-shaped configuration). Specifically, the first support frame 110 may define at least one first opening 117 at the first proximal end, at each of which, a respective first proximal anti-flex feature 113 protruding toward the outside of the first support frame 110 is provided.

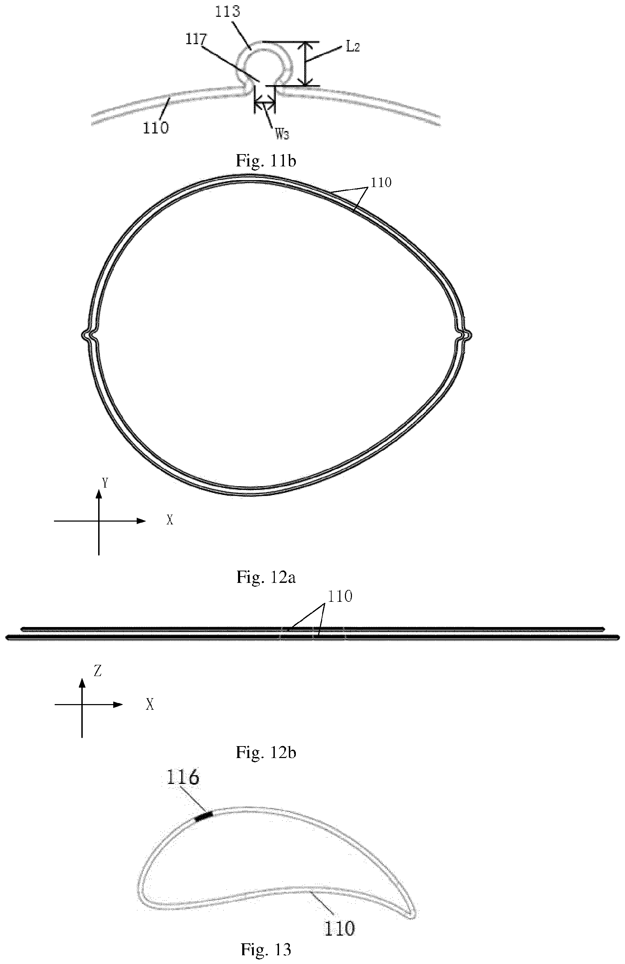

The present embodiment is not exactly limited to any particular form of the first proximal anti-flex feature 113, as long as it can perform the intended function. Generally, the first proximal anti-flex feature 113 may be a V-shaped structure (as shown in Fig. 11a), or a curved structure (as shown in Fig. 11b), or another polygonal structure (not shown). In some embodiments, the first support frame 110 may further define at least one second opening 114 at the first distal end, at each of which, a respective first distal anti-flex feature 115 protruding toward the outside of the first support frame 110 is provided. In the present embodiment, the first distal anti-flex feature 115 may be a V-shaped, curved, or another polygonal structure. In general, only one first proximal anti-flex feature 113 may be provided at the first proximal end of the first support frame 110, and only one first distal anti-flex feature 115 at the first distal end. In this case, both the first proximal anti-flex feature 113 and the first distal anti-flex feature 115 are preferred to be located on a longitudinal axis of symmetry of the teardrop-shaped first support frame 110. As such, when the first support frame 110 is released at the aortic arch in a crimped configuration, once it anchors to the wall of the ascending aorta at the first distal end, a greater expanding force will be generated and then transmitted toward the first proximal end, enhancing the expandability of the entire first support frame 110 (especially the first transitions) and ensuring that the aforementioned coverage area of the embolic diverter 100 is attained. It will be understood that the teardrop-like shape is axis-symmetrical and the axis of symmetry of the teardrop-shaped support frame extends in the first direction. For other possible shapes, the support frame may be similarly configured following the same concept.

Further, with continued reference to Figs. 11a and 11b, a width W₃ of the first opening 117 ranges from 1 mm to 5 mm, and a maximum distance L₂ from the first proximal anti-flex feature 113 to the first opening 117 ranges from 1 mm to 5 mm. Similarly, a width of the second opening 114 ranges from 1 mm to 5 mm, and a maximum distance from the second distal anti-flex feature 115 to the second opening 114 ranges from 1 mm to 5 mm. Preferably, the width W₃ of the first opening 117 ranges from 2 mm to 3 mm, and the maximum distance L₂ from the first proximal anti-flex feature 113 to the first opening 117 ranges from 2 mm to 4 mm. Preferably, the width of the second opening 114 ranges from 2 mm to 3 mm, and the maximum distance from the second distal anti-flex feature 115 to the second opening 114 ranges from 2 mm to 4 mm.

The first filter member 120 may be attached to the first support frame 110 by adhesive bonding, welding, suturing, thermocompression or a laser treatment. The first filter member 120 may be a braided mesh, or a mesh formed by punching a sheet. In general terms, the first filter member 120 is made from a biocompatible material. Specific examples of the material may include nickel-titanium alloys, polymeric materials, inorganic non-metallic materials, etc. Filter openings in the first filter member 120 may be designed as circular openings with an opening size which may range from 50 µm to 250 µm, preferably from 100 µm to 200 µm. A total area of all the filter openings may account for 40-70% of a total area of the first filter member 120. This design ensures that the first filter member 120 can effectively block the passage of clots while not affecting the flow of blood.

In addition, a coating is coated on at least one surface of the first filter member 120 (e.g., its lower surface in the orientation shown in Fig. 12b, i.e., the surface first coming into contact with clots). The coating may be, among others, a heparin coating or an anticoagulant coating, which can prevent accumulation of clots on, and hence occlusion of, the first filter member 120. The coating may be formed by spraying, dipping or another method.

In the present embodiment, the first support frame 110 includes only one closed annular structure. Indeed, the first support frame 110 may also include multiple closed annular structures. As shown in Fig. 12a, in some embodiments, the first support frame 110 includes two closed annular structures which are arranged one inside the other in the same plane. The two annular structures may be interconnected or not, but the first filter member 120 is attached to both of them. In some other embodiments, the first support frame 110 includes two closed annular structures, which may be arranged one above the other in a third direction (i.e., as shown in Fig. 12b). Likewise, the two annular structures may be interconnected or not, and the first filter member 120 is attached to both of them. In some embodiments, the first filter member 120 may consist of multiple layers, and filter openings in different layers may have either the same or different diameters.

As shown in Fig. 13, in another exemplary embodiment of the present application, the first support frame 110 is a three-dimensional structure. In this case, the plane defined by the first support frame 110 may be so curved as to be convex toward the concave surface of the first filter member 120. A projection of the first support frame 110 on an X-Z plane is curved. Moreover, a projection of the first support frame 110 on an X-Y plane may be circular, elliptic, ovate, teardrop or otherwise shaped. This three-dimensional structure enables the first support frame 110 to even better adapt to a varying radius of curvature of the aortic arch.

In the present embodiment, the first support frame 110 is sized substantially the same as the previous embodiment. That is, the length L₁ of the first support frame 110 in the first direction may range from 40 mm to 150 mm, preferably from 60 mm to 120 mm. Additionally, the maximum length of the first support frame 110 in the second direction is between 30 mm and 100 mm. In case of the projection of the first support frame 110 on the X-Y plane being teardrop-shaped, the projection may be divided into a third arc, second transitions and a fourth arc, which smoothly transition from one to another. A diameter of the third arc is smaller than a diameter of the fourth arc. A width W₁ of the third arc may range from 20 mm to 80 mm, and a width W₂ of the fourth arc may range from 30 mm to 120 mm. Preferably, the width W₁ of the third arc ranges from 30 mm to 50 mm, and the width W₂ of the fourth arc ranges from 60 mm to 100 mm.

For details in other building components of the embolic diverter 100 according to the present embodiment, reference can be made to the previous embodiment, and a further description thereof is omitted here.

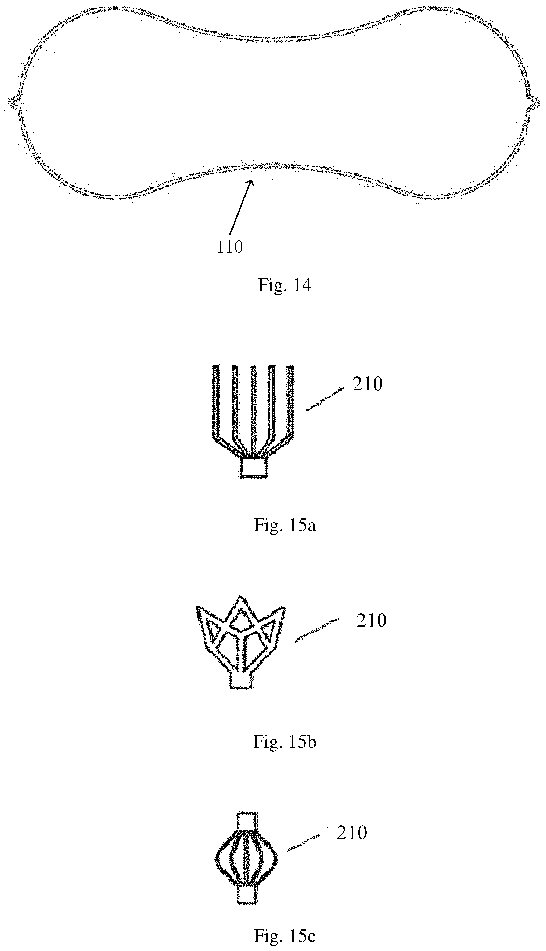

In yet another exemplary embodiment, the first support frame 110 is structured as shown in Fig. 14. The first support frame 110 includes pre-curved first proximal and distal end sections and first and second intermediate sections between the first proximal and distal end sections. The first intermediate section and/or the second intermediate section may be curved so as to convex toward the interior of the first support frame 110. The first support frame 110 of this structure is particularly suited to the case where the aorta gradually narrows from the left common carotid artery 22 to the distal end of the aortic arch 13. When the embolic diverter 100 is released within the aortic arch 13, the first support frame 110 will be compressed by the aortic arch 13, and the recessed portion of the first support frame 110 can impart enhanced outward expandability, which additionally facilitates expansion of the first support frame 110.

Located between the proximal end of the aortic arch and the proximal end of the descending aorta, the embolic collector 200 is able to capture and collect blood clots from blood flowing from the aortic arch toward downstream blood vessels. This can prevent clotting materials that may be produced in the surgical procedure from entering one or more downstream organs and thus possibly cause a complication such as acute kidney injury.

Figs. 15a to 15c are schematic diagrams showing the structure of a second support frame according to an embodiment of the present application. As shown in Figs. 15a to 15c, the embolic collector 200 may include the second support frame 210 and a second filter member 220. In order to facilitate movement of the embolic collector 200 in a blood vessel while avoiding it from causing damage to the wall of the blood vessel, the second support frame 210 may assume a three-dimensional shape inwardly tapered at least at its proximal end, which may resemble, for example, a "claw" (as shown in Fig. 15a), pedals (as shown in Fig. 15b), or a "pumpkin" (as shown in Fig. 15c). The second filter member 220 may be a second filter screen attached to the second support frame 210 and adapted to remove clotting materials such as blood clots from blood flowing through the second filter member 220. Specifically, the second filter member 220 may cover, for example, at least part of the second support frame 210 in order to remove blood clots from blood flowing through the embolic collector 200. The second filter member 220 may be attached to the second support frame 210 on one side thereof in such a manner that peripheral edges of the second filter member 220 are tied to the second support frame 210. The second filter member 220 may be either elastic or not. Optionally, a surface of the second filter member 220 may be coated with a coating. The coating may be heparin, an anticoagulant, etc. It can avoid accumulation of blood clots that may be produced in the surgical procedure on, and hence occlusion of, the second filter member 220. Alternatively, the second filter member 220 may be fabricated from an anticoagulant material with anti-clotting or similar properties.

The portion of the second support frame 210 covered by the second filter member 220 is generally funnel-shaped, and the push tube 300 is inserted through the second support frame 210 of the embolic collector 200 so as to exit the embolic collector at an opening of the funnel and is attached thereto at the opening. That is, the second support frame 210 is disposed over the push tube 300. The attachment of the push tube 300 to the second support frame 210 may be accomplished, for example, by laser, pressing or heat setting, or chemically (e.g., using glue). The second support frame 210 is adapted to support the second filter member 220 and define a circumferential coverage area thereof so that a radial cross section upstream of downstream blood vessels is completely spanned.

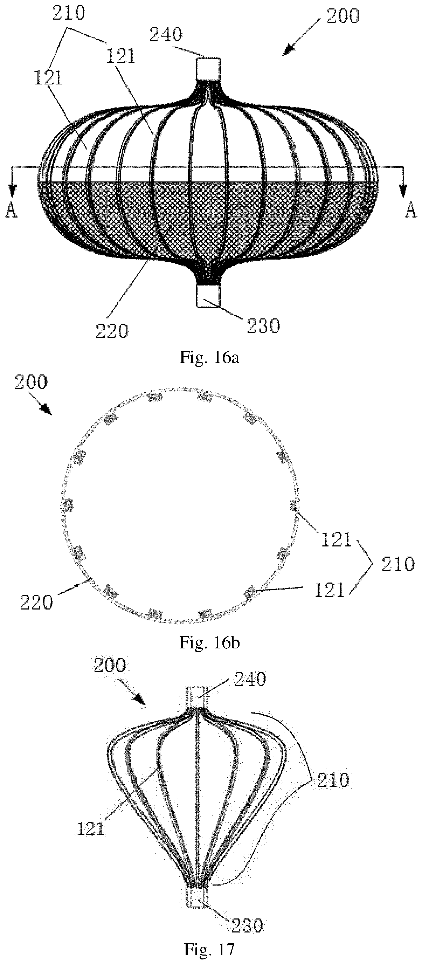

Fig. 16a shows the embolic collector 200 according to another embodiment of the present application, which includes a proximal connection 230, the second support frame 210, the second filter member 220 and a distal connection 240. The second support frame 210 is adapted to support the second filter member 220 so that the second filter member 220 partially fits against the inner wall of the blood vessel and is able to remove clotting materials.

With continued reference to Fig. 16a, the second support frame 210 includes multiple support struts 121 spaced apart from one another around an axis. At least some of the support struts 121 are curved to define a concave plane facing the axis. Each of the support struts 121 has a first proximal end and a first distal end opposing the first proximal end along the direction of the axis. All the support struts 121 are gathered by the proximal connection 230 at their first proximal ends and by the distal connection 240 at their first distal ends. In other words, each support strut 121 is spaced from the axis by a distance that first increases and then decreases in the direction from the first proximal end to the first distal end. As a result, the second support frame 210 appears wide in the middle and narrow at both ends.

The second filter member 220 is an umbrella-shaped filter screen having a second proximal end and an opposing second distal end. The second filter member 220 is provided on the second support frame 210, with the second proximal end being situated at the first proximal ends of the support struts 121 (i.e., at the proximal connection 230). The second distal end is disposed on the second support frame 210 so as to define an opening allowing clots to enter the second filter member 220 and thus be trapped therein.

It would be appreciated that, in the present embodiment, the aforementioned axis is of the embolic collector 200. As used hereinafter, the terms "axial", "circumferential" and "radial" refer to directions parallel to, about and perpendicular to the axis, respectively.

The embolic collector 200 is deployed in the blood vessel so that the second distal end of the second filter member 220 serves as an inflow end of the embolic collector 200 and the second proximal end of the second filter member 220 serves as an outflow end of the embolic collector 200.

In the present embodiment, the second support frame 210 consists essentially of the multiple support struts 121, which are arranged in a radiating pattern and are not interconnected by any circumferential connection. Therefore, when attached to the support struts 121, the second filter member 220 may be recessed toward the axis between adjacent support struts 121. Alternatively, after the second filter member 220 is attached to the support struts 121, it may be recessed between adjacent support struts 121 when urged toward the axis. In some embodiments, two adjacent ones of the support struts 121 may be spaced apart by a gap that is wider than a gap between any other two adjacent ones of the second support struts 121. In this case, when urged toward the axis, the second filter member 220 may be recessed between said two adjacent second support struts. In particular, when the embolic collector 200 is deployed in the blood vessel, even when the support struts 121 are densely arranged (i.e., with small gaps between adjacent support struts), the passage of an instrument delivery tube (not shown) will be still allowed when it presses against the support struts 121 to expand the gaps between them and thus enlarge the recesses. In other words, the second support frame 210 will neither affect the insertion or withdrawal of a surgical instrument into or from the blood vessel, nor make the passage of the surgical instrument difficult or impossible.

The number of the support strut 121 may range from 4 to 25, more preferably from 12 to 18. All the support struts 121 may be equally wide, or at least some of them have different widths (as shown in Fig. 16b). In the case of at least some of the support struts 121 having different widths, a width ratio of the widest support strut 121 to the narrowest support strut 121 may range from 1.2 to 2. The different widths of the support struts 121 in the second support frame 210 can provide uneven support to the embolic collector 200 deployed in the blood vessel, which allows easier passage of the instrument delivery tube through the embolic collector 200. It would be appreciated that the term "width" refers to a circumferential size of each support strut 121 in the second support frame 210.

In the present embodiment, the second support frame 210 may assume any of various shapes such as spherical, olive-like and double-tapered. A first location may be equally distant from the first proximal and distal ends (i.e., located at the middle of the second support frame 210, as shown in Fig. 16a) or not (i.e., not located at the middle of the second support frame 210, as shown in Fig. 17). In the case of that the first location is not in the middle of the second support frame 210, the distance from the first location to the first distal end is smaller than the distance from the first location to the first proximal end, or, the distance from the first location to the first distal end is greater than the distance from the first location to the first proximal end.

Preferably, the second filter member 220 is attached to the second support frame 210 so that the second distal end is flush with the first location and circumferentially surrounds the second support frame 210. In this way, the second filter member 220 can fit against the inner wall of the blood vessel at the second distal end to maximize the ability of the second filter member 220 to capture clots and avoid any clot from being stuck between the embolic collector 200 and the inner wall of the blood vessel. Alternatively, the second filter member 220 is attached to the second support frame 210 so that the second distal end is located between the first location and the first distal ends of the support struts 121 and circumferentially surrounds the second support frame 210.

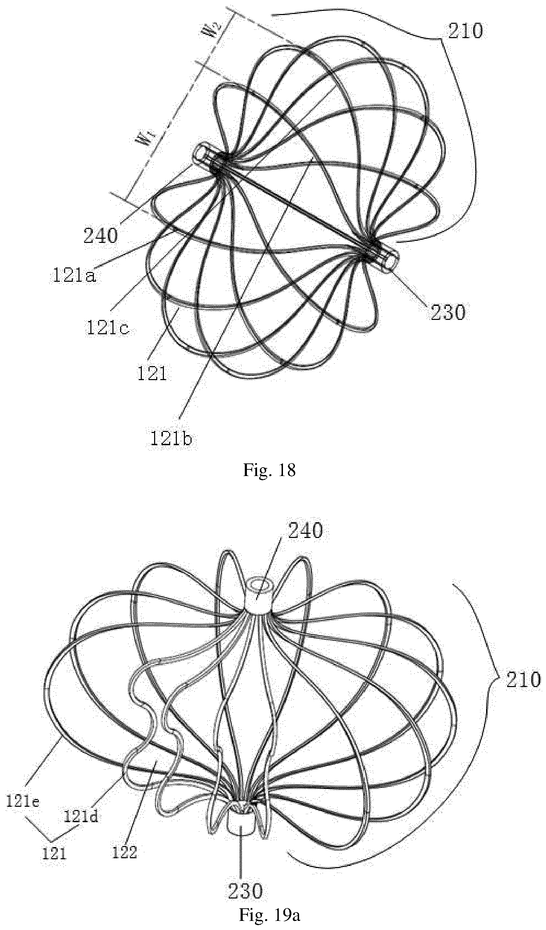

Optionally, the support struts 121 may be arranged around the axis either evenly or not as shown in Fig. 18. With particular reference to Fig. 18, there are three adjacent support struts 121 in the second support frame 210 of the embolic collector 200, referred hereinafter respectively as a third support strut 121a, a fourth support strut 121b and a fifth support strut 121c. The third support strut 121a is spaced apart from the fourth support strut 121b by a spacing W1, and the fourth support strut 121b from the fifth support strut 121c by a spacing W2. Preferably, W2 is unequal to W1. This arrangement is also advantageous in forming the above-discussed recesses that can facilitate passage of the instrument delivery tube through the embolic collector 200, in particular when the embolic collector 200 is positioned in the descending aorta 12 and the instrument delivery tube is advanced on the top side of the inner wall of the descending aorta 12. In the case, the two farther spaced support struts 121 may be positioned at said top side (e.g., when W2<W1, the third support strut 121a and the fourth support strut 121b may be placed in closer proximity to the top side). In this way, passage of the instrument delivery tube can be made easier, and a larger portion of the second filter member 220 can be left between the two farther spaced support struts 121, which can more tightly wrap the instrument delivery tube, avoiding peripheral escape of clots.

In general cases, the embolic collector 200 is crimped within a delivery device and advanced therein to a target site in the blood vessel. Therefore, the second support frame 210 may be made of an elastic material. In some embodiments, the second support frame 210 may be formed by laser cutting a tube or sheet. In some other embodiments, the second support frame 210 may be braided from one or more wires.

The second filter member 220 may be attached to at least some of the support struts 121 in the second support frame 210, for example, by adhesive bonding, welding, thermocompression, suturing or a laser treatment. The second filter member 220 may be formed of a biocompatible material such as a nickel-titanium alloy, a polymer material or an inorganic non-metallic material. The second filter member 220 may be formed either by punching a sheet of the biocompatible material, or braiding one or more wires of the biocompatible material into a mesh. Depending on the material used, the second filter member 220 may be either elastic or not. Filter openings in the second filter member 220 may be circular or otherwise shaped. In the former case, the filter openings may have a diameter ranging from 30 µm to 250 µm, preferably from 100 µm to 200 µm. A total area of the filter openings in the second filter member 220 may account for 40-70% of a total area of the second filter member 220. In addition, the second filter member 220 may consist either of a single filter screen, or of a stack of multiple filter screens with filter openings of the same or different sizes.

Further, a coating (not shown) is provided on at least one surface of the second filter member 220, e.g., the surface thereof facing the inflow end. The coating may be, among others, a heparin coating or an anticoagulant coating, which can prevent accumulation of clots on, and hence occlusion of, the second filter member 220. The coating may be formed by spraying, dipping or another method.

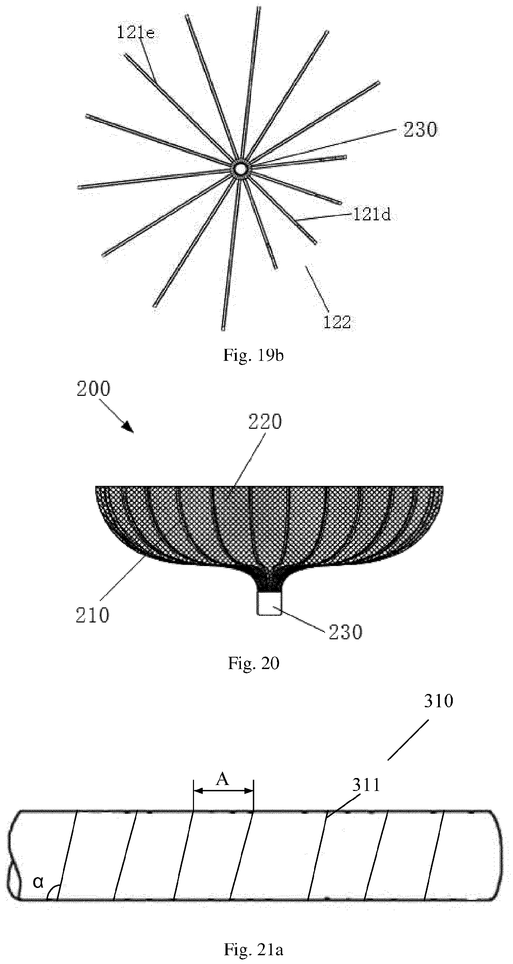

Figs. 19a and 19b are schematic diagrams showing the structure of a second embodiment of the embolic collector 200 according to the present application. This embodiment differs from the first embodiment in how one or more recesses are formed. Specifically, the second support frame 210 includes at least one first support strut 121d and several second support struts 121e. In case of two or more first support struts 121d being included, these first support struts 121d are arranged to be adjacent to each other. Each first support strut 121d has a recessed portion that is convex toward the axis. When the second filter member 220 is attached to the first support struts 121d, a recess 122 is formed over the recessed portions. Similarly, this embolic collector 200 may be deployed in the blood vessel with the recess 122 facing the top side of the inner wall of the blood vessel and delimiting an instrument channel therewith, through which the instrument delivery tube can pass. This design is particularly suited to the case where there are a relatively large number of (e.g., 15 or more) support struts 121 in the second support frame 210.

In the present embodiment, at the first location, the second support strut 121e may be arranged on the same arc having a diameter that may be configured taking into account the diameter of the second support frame 210 in the first embodiment, as long as when deployed in the blood vessel, the second support frame 210 can support the second filter member 220 and fit part of it against the inner wall of the blood vessel to remove clotting materials 300.

Fig. 20 shows a schematic diagram showing the structure of a third embodiment of the embolic collector 200 according to the present application. In the present embodiment, the embolic collector 200 does not include the distal connection. More particularly, the support struts 121 may be either curved or straight and each be spaced from the axis by a distance gradually increasing in the direction from the first proximal end to the first distal end. In other words, in the present embodiment, when the second support frame 210 has a circular cross section perpendicular to the axis, the second support frame 210 has a diameter maximum at the first distal ends of the support strut 121. Thus, the first location is situated at the first distal ends to which the second distal end of the second filter member 220 is attached. It would be appreciated that, here, the first distal ends may refer to either end points or end portions each having a certain length (in the latter case, the portions are straight).

It is a second object of embodiments of the present application to provide an embolic protection system adapted to be implanted into the aorta prior to or during a transcatheter procedure in which clotting materials may be produced, such as transcatheter mitral valve replacement (TMVR) or left atrial appendage closure, in order to collect such clots. Referring to Fig. 16, the embolic protection system includes a delivery device, an embolic diverter 100 and an embolic collector 200 according to any preceding embodiment. The delivery device has a fourth proximal end and an opposing fourth distal end. The embolic diverter 100 is coupled to the delivery device and includes a second support member and a second filter member disposed on the second support member. The embolic diverter 100 is configured to fit against the inner wall of the aortic arch 210 in an expanded configuration so as to cover the branch blood vessels arising from the aortic arch 210. The embolic collector 200 is coupled to the delivery device between the embolic diverter 100 and the fourth proximal end. The embolic collector 200 is configured to extend over the wall of the descending aorta 12 in an expanded configuration, and the second filter member 220 is open at a second distal end thereof toward the embolic diverter 100. The delivery device is adapted to deliver the embolic diverter 100 and the embolic collector into the blood vessel. The push tube 300 defines a lumen extending through the push tube 300 along the direction of extension thereof. The lumen may be adapted to allow the passage therethrough of an associated guidewire for use in, e.g., a TAVR surgical procedure so that the guidewire is able to guide the push tube 300 to a predetermined site such as the aortic arch 13. The push tube 300 may be formed of a metal (e.g., stainless steel or a nickel-titanium alloy), a polymer, or metal-polymer composite material. The push tube 300 may have a constant diameter from the proximal end to the distal end. Alternatively, the push tube 300 may have a varying diameter that is greater at the proximal end and smaller at the distal end. Further, the push tube 300 may receive a special structuring process such as setting or pressing at the distal or proximal end, which makes the push tube 300 bendable at various angles at said end. In this way, when a medical instrument is advanced over the guidewire to the aortic arch, it can actively come into conformity with the anatomy of the aortic arch. As a result, the instrument will not flip over and can be released stably. The push tube 300 may be coupled at the distal end to either or both of the distal and proximal ends of the first support frame 110 or first filter member 120. Preferably, the push tube 300 is coupled at the distal end to both of the distal and proximal ends of the first support frame 110 or first filter member 120. This can impart good wall conformity to the push tube 300 that is supported by the guidewire while ensuring desirable wall conformity of both ends of the first support frame 110.

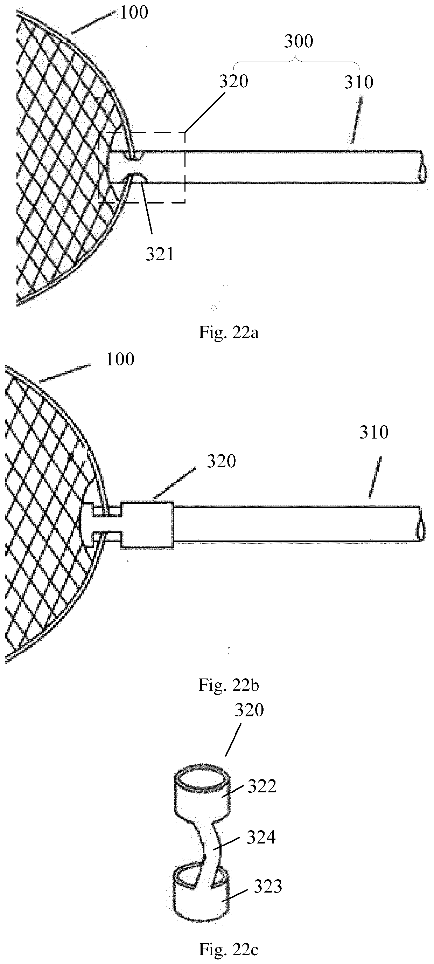

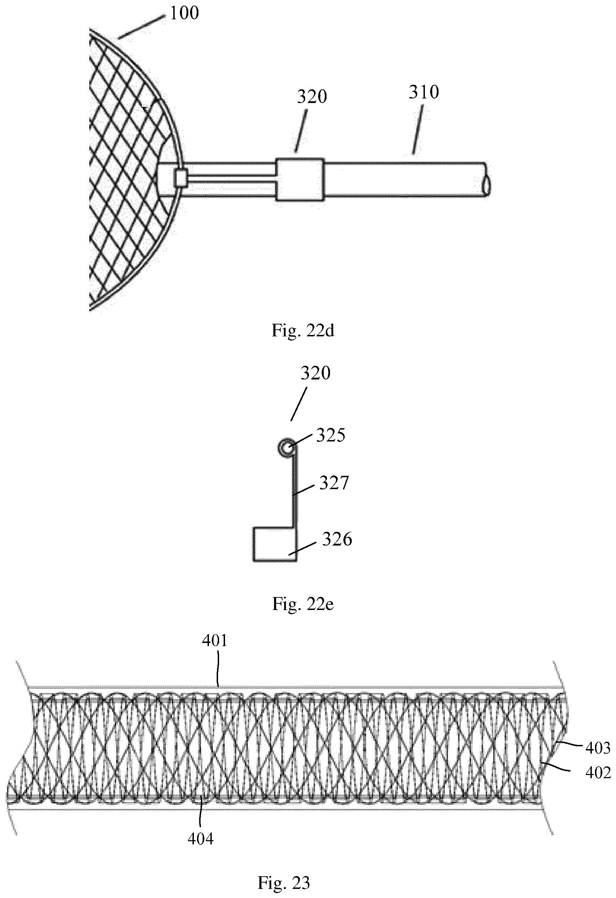

Fig. 22a is a schematic diagram showing the structure of a coupling element according to an embodiment of the present application. As shown in Fig. 22a, the push tube 300 includes a tubular body 310 and, both disposed on the tubular body 310, a first coupling element 320 and a second coupling element. The first coupling element 320 is coupled to the tubular body 310. Specifically, the tubular body 310 may be coupled to the first coupling element 320, for example, by laser, pressing or heat setting, or chemically (e.g., using glue) so that the first coupling element 320 couples the embolic diverter 100 to the tubular body 310. The first coupling element 320 is proximal to the distal end of the push tube 300, and the push tube 300 is coupled to the embolic diverter 100 by the first coupling element 320. The tubular body 310 further includes a distal cut hypotube section, which can be shaped by the guidewire in the push tube 300 into conformity with a patient's aortic arch anatomy, imparting better wall conformity to the first support frame 110 and/or the first filter member 120. The second coupling element is disposed between the first coupling element 320 and a proximal end of the tubular body 310. The embolic collector 200 can be coupled to the push tube 300 by the second coupling element. The second coupling element may include a groove running circumferentially around the tubular body 310, and the embolic collector 200 may be disposed over the second coupling element at the groove and thus coupled to the push tube 300.

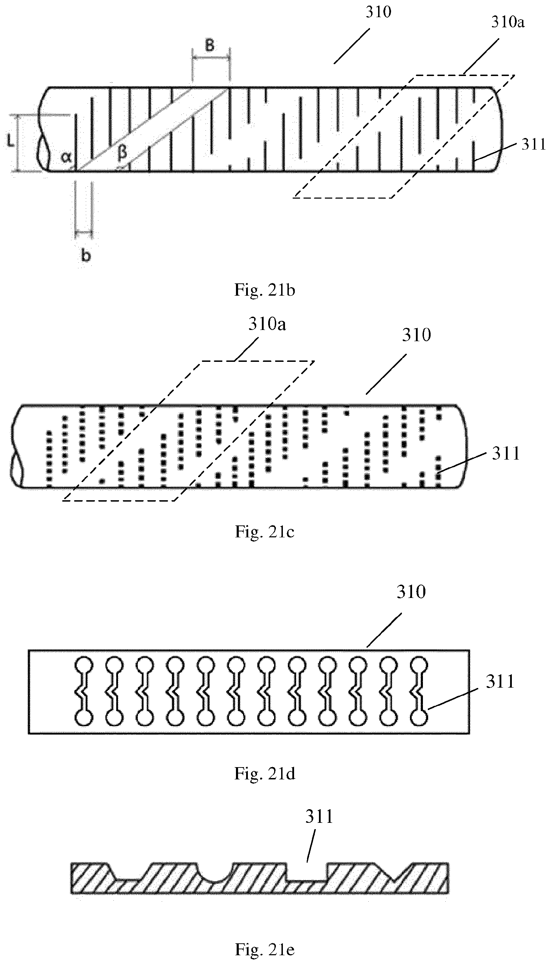

Figs. 21a to 21d are schematic diagrams showing the structures of several variants of the cut hypotube section of the tubular body in the present embodiment. As shown in Figs. 21a to 21d, the cut hypotube section has a length of from 80 mm to 350 mm, preferably from 100 mm to 230 mm, along an axis of the tubular body 310.

As shown in Fig. 21a, the cut hypotube section includes a plurality of cut units 311 which are arranged along the axis of the tubular body 310 into a helical pattern. The cut units 311 may each include at least one groove. In case of the cut units 311 each including at least two grooves, these grooves are spaced apart from each other in the direction of extension of the cut unit 311. A pitch A of the cut units 311 (i.e., a distance between any two adjacent ones of the cut units 311 along the axis of the tubular body 310) ranges, for example, from 0.7 mm to 2.5 mm, preferably, for example, from 1.2 mm to 2.2 mm. An angle α of each cut unit 311 (i.e., an angle formed by the specific cut unit 311 with the axis of the tubular body 310) ranges, for example, from 0° to 90°, preferably, for example, from 60° to 90°.

As shown in Figs. 21b and 21d, the cut hypotube section includes a plurality of cut portions 310a spaced apart along the axis of the tubular body 310, each containing at least one cut unit 311 circumferentially spaced apart around the tubular body 310. A pitch B of the cut portions 310a (i.e., a distance between any two adjacent ones of the cut portions 310a along the direction of extension of the tubular body 310) ranges, for example, from 0.7 mm to 2.5 mm, preferably, for example, from 1.2 mm to 2.2 mm. An angle β of each cut portion 310a (i.e., an angle formed by the specific cut portion 310a with the direction of extension of the tubular body 310) ranges, for example, from 0° to 180°. When the angle β of the cut portions 310a is 90°, their cut units 311 are arranged into discontinuous annular bands on the push tube 300, which are spaced apart along the axis of the push tube 300. When the angle β of the cut portions 310a is 0° or 180°, the cut portions 310a extend in parallel to the axis of the tubular body 310. Preferably, the angle β of the cut portions 310a ranges, for example, from 40° to 65°, or from 115° to 140°. In other implementations, the cut hypotube section also includes a plurality of cut portions spaced apart circumferentially around the tubular body, each containing at least one cut unit spaced apart along the axis of the tubular body. Each of the cut portions may be structured the same as any of the above-described cut portions 310a spaced apart along the axis of the tubular body 310.

An angle α of each cut unit 311 with the axis of the tubular body 310 ranges, for example, from 0° from 90°, preferably, for example, from 60° to 90°. A width of each cut unit 311 (i.e., its groove(s)), i.e., its size in the direction perpendicular to its direction of extension may range from 0.01 mm to 2.00 mm, preferably from 0.02 mm to 1.50 mm. A pitch b of the cut units 311, i.e., a distance between any two adjacent ones of the cut units 311, may range from 0.05 mm to 0.50 mm, preferably from 0.05 mm to 0.30 mm. A length L of each cut unit 311, which is defined as its length running on an outer surface of the tubular body, is within a range determined by a circumferential perimeter M of the tubular body 310. Specifically, a ratio P of the length L of each cut unit 311 to the circumferential perimeter M of the tubular body 310 (i.e., P=L/M) ranges, for example, from 0.08 to 0.90, preferably, for example, from 0.10 to 0.85. At least one of the angle α of the cut units 311, the angle β of the cut portions 310a, the width a of the cut units 311, the pitch b of the cut units 311, the pitch A of the cut units 311 and the pitch B of the cut portions 310a may vary gradually or in steps so that the tubular body 310 may have a hardness gradient or different hardnesses at different portions. As a result, the push tube can not only be shaped by the internal guidewire, but will also not experience any significant or strong stress concentration, which may lead to excessive flexure of the push tube, when it is pushed or pulled or during its transmission of a pushing or pulling force.

As shown in Fig. 21e, in order to further enhance hardness variability of the tubular body 310, the cut units 311 may have cross-sectional shapes (perpendicular to their extension) including, but not limited to, trapezoidal, semi-circular, rectangular, triangular, etc.

Each cut unit 311 includes at least one groove. In case of each cut unit 311 including at least two grooves, these grooves are spaced apart along the direction of extension of the specific cut unit. Each groove may extend through the tubular body 310 from its exterior to the lumen, or not. As shown in Fig. 21d, each cut unit 311 may further include two circular recesses at its opposite ends and an elongate groove connecting the two circular recesses. The elongate groove may further include at least one V-shaped groove portion. The circular recesses may be brought into communication with the elongate groove or not.

Fig. 22a is a schematic diagram showing the structure of a first variant of the coupling element according to the present application. As shown in Fig. 22a, the first coupling element 320 extends in the same direction as the axis of the tubular body 310 and is integrally formed with the tubular body 310. The first coupling element 320 has a through hole 321, which extends through the tubular body 310 in a direction perpendicular to the axis of the tubular body 310 and has a diameter smaller than that of a cross section of the first coupling element 320 (i.e., its cross section perpendicular to the axis of the tubular body 310). The first support frame 110 is, for example, partially passed through the through hole 321, thereby coupling the embolic diverter 100 to the push tube 300. The through hole 321 defines two opposing openings, referred to hereinafter respectively as a first opening and a second opening. A maximum distance between the first and second openings (i.e., a maximum length of the through hole) ranges, for example, from 2.5 mm to 7 mm, preferably from 3 mm to 5 mm. A minimum distance between the first and second openings (i.e., a minimum length of the through hole) ranges, for example, from 0.2 mm to 1 mm.

Figs. 22b to 22c are schematic diagrams showing the structure of a second variant of the coupling element according to the present application. As shown in Figs. 22b to 22c, in this case, the first coupling element 320 is H-shaped and extends in the same direction as the axis of the tubular body 310. The first coupling element 320 includes a first tubular element 322, a first connecting bar 324 and a second tubular element 323, which are connected together sequentially in the direction from the distal to proximal end of the tubular body 310. The first tubular element 322, the first connecting bar 324 and the second tubular element 323 are arranged coaxially with and disposed over the tubular body 310. The first tubular element 322 is tied to the second tubular element 323 by the first connecting bar 324. Specifically, the first connecting bar 324 is connected, for example, distally to the first tubular element 322 at a proximal opening thereof and, for example, proximally to the second tubular element 323 at a distal opening thereof. The proximal connection of the first connecting bar 324 to the second tubular element 323 at the distal opening thereof may be accomplished, for example, by laser, pressing or heat setting, or chemically (e.g., using glue). The first connecting bar 324 is, for example, an elongate bar that may be either flat or convex. Preferably, the first connecting bar 324 may be an elongate bar that is convex away from the tubular body 310 so that a through hole 321 is delimited between the first connecting bar and the tubular body to help relieve stress from the first tubular element 322 and the second tubular element 323. The first support frame 110 and/or the first filter member 120 may be partially passed through the first tubular element 322, with the second tubular element 323 being proximally coupled to the distal end of the tubular body 310. Specifically, for example, an inner diameter of the second tubular element 323 is greater than a distal outer diameter of the tubular body 310 to allow the second tubular element 323 to be proximally disposed over the distal end of the tubular body 310, coupling the embolic diverter 100 to the push tube 300. A length of the first tubular element 322 (along its extension) may be equal to a length of the second tubular element 323, or not. The length of the first tubular element 322 and that of the second tubular element 323 may both range from 0.5 mm to 5 mm, preferably from 1 mm to 3 mm. The first tubular element 322 and the second tubular element 323 may be spaced apart by a distance ranging, for example, from 2.5 mm to 7 mm, preferably from 3 mm to 5 mm. A width of the first connecting bar 324 (i.e., its size in the direction perpendicular to the axis of the tubular body) ranges, for example, from 0.2 mm to 1 mm. A height (of convexity) of the first connecting bar 324 ranges, for example, from 0.5 mm to 3 mm, preferably, for example, from 0.8 mm to 2.5 mm.

Figs. 22d to 22e are schematic diagrams showing the structure of a third variant of the coupling element according to the present application. As shown in Figs. 22d to 22e, in this case, the first coupling element 320 is H-shaped and includes a third tubular element 325, a second connecting bar 327 and a fourth tubular element 326, which are connected together sequentially in the direction from the distal to proximal end of the tubular body 310. The fourth tubular element 326 is arranged coaxially with and disposed over the tubular body 310. The third tubular element 325 is tired to the fourth tubular element 326 by the second connecting bar 327. The third tubular element 325 has an axis perpendicular to the axis of the tubular body 310 and a lumen defining a through hole 321. The third tubular element 325 is coupled to the fourth tubular element 326 by the second connecting bar 327. Specifically, the second connecting bar 327 is connected proximally to a distal end of the fourth tubular element 326 and distally to an outer wall of the third tubular element 325 so that the fourth tubular element 326 extends in a direction perpendicular to the axis of the tubular body 310. The distal connection of the second connecting bar 327 to the outer wall of the third tubular element 325 may be accomplished, for example, by laser, pressing or heat setting, or chemically (e.g., using glue). The first support frame 110 or the first filter member 120 is partially passed through the third tubular element 325, and an inner diameter of the fourth tubular element 326 is greater than the outer diameter of the tubular body 310 to allow the fourth tubular element 326 to be disposed over the distal end of the tubular body 310. The third tubular element 325 may have an outer diameter ranging from 1 mm to 3 mm and a length ranging from 0.6mm to 1.5 mm. The fourth tubular element 326 may have a length ranging from 0.5 mm to 5 mm, preferably from 1 mm to 3 mm. The second connecting bar 327 is, for example, a straight bar. A width of the second connecting bar 327 may range from 0.2 mm to 1 mm, and a length of the second connecting bar 327 may range from 1 mm to 6mm, preferably from 2 mm to 4 mm.

The first coupling element 320 may be made of a metallic, polymeric or ceramic material, which can facilitate an operator's manipulation of the embolic protection device (in particular, including release and movement control) and enables the first coupling element 320 to provide stronger pushing and pulling forces. Thus, the operator can rapidly change the position of or pivot the first support frame 110 with higher angular positioning accuracy, rotatability of the first support frame 110 both within 180° about the direction of extension of the through hole 321 and within 360°about the axis of the push tube 300 and enhanced wall conformity of the embolic diverter 100.

The present application further provides a medical catheter, which can be used either as a delivery sheath or for other medical applications. The medical catheter is, for example, a hollow tube. In one embodiment, the medical catheter is implemented as, for example, a delivery sheath used for providing a path for delivery, release, retrieval or the like of an intravascular interventional device. Fig. 23 is a schematic perspective view of the delivery sheath according to an embodiment of the present application. The delivery sheath 400 is a hollow tubular structure and defines a lumen extending through the delivery sheath 400 along the direction of extension thereof and configured to accommodate therein the embolic diverter 100, the embolic collector 200, both in a crimped configuration, and the push tube 300. In order to facilitate introduction of the interventional device into a blood vessel during a cardiac or aortic procedure, a suitable diameter range for the delivery sheath 400 may include, but is not limited to, from 6F to 12F. In other embodiments, the diameter of the delivery sheath 400 may vary as actually needed.

The delivery sheath 400 includes at least two axially-extending segments. As a specific example, the delivery sheath 400 includes two axially-extending segments, which are an axially distal segment and an axially opposing, proximal segment. Alternatively, the delivery sheath 400 includes at least three axially-extending segments, which are an axially distal segment, an axially opposing, proximal segment and at least one intermediate segment between the distal and proximal segments. The distal segment may be inwardly tapered. That is, a diameter of the distal segment may gradually decrease in the direction away from the proximal segment. This allows the delivery sheath 400 to more easily enter the blood vessel without causing damage to its wall. Tensile strength of the delivery sheath 400 may be up to 60 N to 120 N, which can ensure a sufficient pushing force as required by release of the intravascular interventional device, as well as desirable stability and insignificant elongation of the intravascular interventional device. It is to be noted that the term "proximal" refers to an end closer to the operator and the term "distal" refers to an end farther away from the operator.

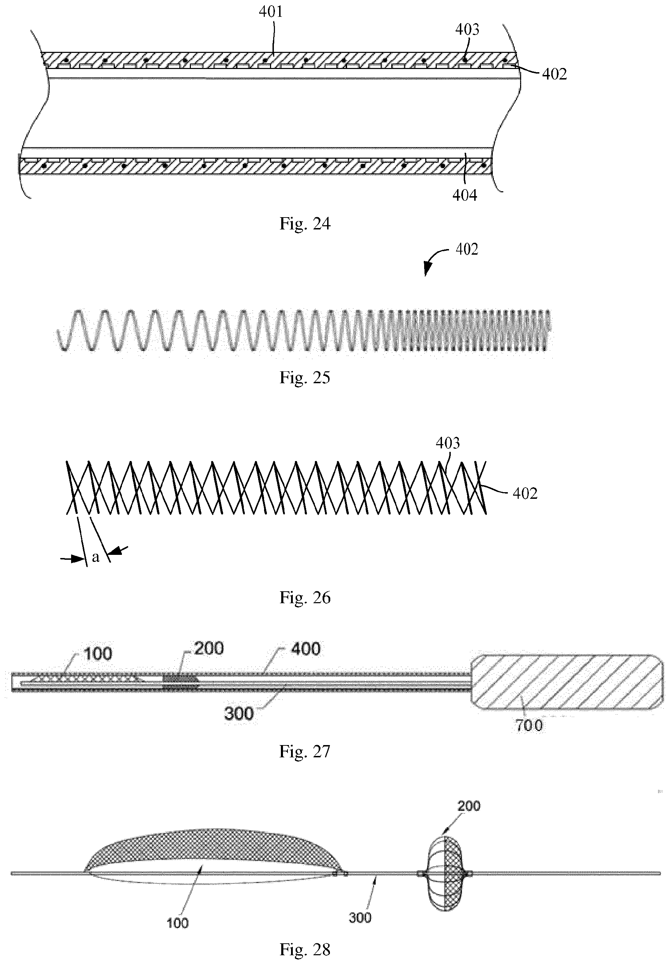

Fig. 24 is an axial cross-sectional view of the delivery sheath 400 according to an embodiment of the present application. As shown in Fig. 24, with combined reference to Fig. 23, the delivery sheath 400 includes an inner layer 404, a helical layer 402 and a braided layer 403, the latter two of which are both disposed externally around the inner layer 404. The inner layer 404 may be disposed externally around the helical layer 402, and the helical layer 402 around the braided layer 403. In other embodiments, the inner layer 404 may be disposed externally around the braided layer 403, and the braided layer 403 around the helical layer 402. The delivery sheath 400 may further include an outer layer 401 disposed externally around the inner layer 404, in which both the helical layer 402 and the braided layer 403 are buried. In other embodiments, the helical layer 402 and the braided layer 403 may be both disposed between the inner layer 404 and the outer layer 401.

The outer layer 401 may be made from any of a polyether block amide (Pebax) resin, a nylon resin, a polyimide resin or a fluoropolymer resin such as polytetrafluoroethylene (PTFE). Preferably, the outer layer 401 is made from a Pebax resin, because it can impart increased compliance and resistance to chemicals and corrosion by salts or sulfuric acid to the outer layer 401, allow the outer layer 401 to encounter reduced resistance in blood and improve its biocompatibility. Pebax resins with various hardnesses are available, such as those with Shore hardnesses in the range of from 25 D to 74 D. Depending on the requirements of particular applications, the outer layer 401 may be entirely made of the same Pebax resin with a desired hardness, or different portions of the outer layer 401 may be made of Pebax resins with different hardnesses. For example, a proximal end portion of the outer layer 401 may be made of a Pebax resin with a high hardness, while a distal end portion thereof may be made of a Pebax resin with a low hardness. As a result, the delivery sheath 400 will exhibit excellent pushability and strength at the proximal end and excellent compliance and flexibility at the distal end.

In order to enable the delivery sheath 400 to readily enter the blood vessel, the outer layer 401 is optionally provided with a coating on its outer wall, such as a hydrophilic coating, a hydrophobic coating or a self-lubricating fluoroplastic.

The inner layer 404 defines a hollow lumen adapted to accommodate therein the aforementioned intravascular interventional device. The inner layer 404 may be made of polytetrafluoroethylene (PTFE), which has good chemical stability, corrosion resistance, sealing ability, high lubricity, non-adhesiveness and biocompatibility. Using PTFE as the material of the inner layer 404 allows the intravascular interventional device to encounter less resistance and be more accurately and controllably positioned during its release and can reduce resistance encountered by the delivery sheath 400 during its entry into the blood vessel. Moreover, the inner layer 404 is enabled to provide advantages, including exhibiting a low coefficient of friction and having good biocompatibility, which allows it to not cause rejection by or physiological side effects on the human body.

The braided layer 403 is, for example, a mesh layer which enables the delivery sheath 400 to have increased radial and axial strength and flexibility. The braided layer 403 may be composed of, for example, several interlaced first filaments. Specifically, the braided layer 403 may be made either by helically twisting the first filaments into a cord and then interlacing the cord, or by directly interlacing the individual first filaments. As such, it is ensured that the braided layer 403 possesses good tensile strength, comparable mechanical properties to those of cables, desirable flexibility and satisfactory fatigue resistance after repeated twists and bends and is thus suited to tortuous blood vessels. Preferably, the braided layer 403 is formed of, for example, 4 to 64 interlaced first filaments. The first filaments are interlaced, for example, in a "1 on 1" or "2 on 2" pattern to form the braided layer 403. Of course, other interlacing patterns can also be employed to form the braided layer 403, and a suitable pattern can be chosen according to the actual need. The first filaments may be so interlaced that the resulting braided layer 403 has uniform or non-uniform density of openings. As a particular example, the density of openings in the braided layer 403 may gradually increase from a proximal end of the delivery sheath 400 to a distal end thereof. Alternatively, the density of openings in the braided layer 403 may increase from the proximal to distal end of the delivery sheath 400 in steps corresponding to the aforementioned respective segments. The density of openings formed by the first filaments may vary depending on an intended load carrying capability of the delivery sheath 400 as well as on the material of the first filaments. Preferably, the density of openings formed by the first filaments gradually increases from the proximal to distal end and ranges from 20 PPI to 120 PPI, thus enabling the delivery sheath 400 to have desirable supporting ability, compliance and pushability and allowing the braided layer 403 to bear the majority of axial tension during release of the intravascular interventional device. A diameter of the first filaments ranges from 0.012 mm to 0.1 mm, in particular from 0.012 mm to 0.05 mm. Preferably, the diameter of the first filaments ranges from 0.012 mm to 0.025 mm. In order for the braided layer 403 to have increased deformation resistance and improved adhesion to the outer layer 401, and in order for the delivery sheath 400 to have a reduced wall thickness, which allows an expanded lumen while not creating additional pressure on the blood vessel, the first filaments may be made of aramid (AF), polyester (PES), polyethylene terephthalate (PET), polypropylene (PP), nylon, liquid crystal polymer (LCP), polyamide or another polymeric material, or a combination thereof.

Fig. 25 is a schematic diagram showing the structure of the helical layer according to an embodiment of the present application. As shown in Fig. 25, the helical layer 402 is, for example, helical around an axis of the delivery sheath 400. This enables the delivery sheath 400 to have increased axial strength and compliance. The helical layer 402 may be formed of a number of second filaments that are helically coiled along said axis. Specifically, the helical layer 402 may be made either by helically twisting the second filaments into a cord and then helically coiling the cord along the axis, or by directly helically coiling the individual second filaments along the axis. The helical layer 402 may also be a laser-cut tube. The helical layer 402 may be so formed that the second filaments define openings distributed with either a uniform or non-uniform density. The density of openings in the helical layer 402 may gradually decrease from the proximal to distal end of the delivery sheath 400. Alternatively, the density of openings in the helical layer 402 may decrease from the proximal to distal end of the delivery sheath 400 in steps corresponding to the aforementioned respective segments.

The delivery sheath 400 may include at least three axially-extending segments, which are an axially distal segment, an axially opposing, proximal segment and at least one intermediate segment between the distal and proximal segments. The density of openings in the helical layer 402 may range from 20 PPI to 55 PPI, preferably from 20 PPI to 35 PPI, at the distal segment. The density of openings in the helical layer 402 may range from 35 PPI to 100 PPI, preferably from 35 PPI to 45 PPI, at the intermediate segment. The density of openings in the helical layer 402 may range from 45 PPI to 150 PPI, preferably from 45 PPI to 60 PPI, at the proximal segment. The second filaments may have a diameter, for example, between 0.025 mm and 0.3 mm, and may be, for example, round or flat.

The second filaments may form an angle of 45°-90° with the axis of the delivery sheath 400. Preferably, the angle between the second filaments and the axis of the delivery sheath 400 ranges from 60° to 80° so that the helical layer 402 can bear radial pressure exerted on the delivery sheath 400 by the intravascular interventional device during its expansion while maintaining very strong kink resistance, which can avoid physical damage occurring at a bend of the blood vessel or as a consequence of an excessive manipulating force, such as deformation or flexture. The second filaments may be formed of a metal such as stainless steel or a nickel-titanium alloy, or a polymeric material such as polyetheretherketone (PEEK) or high density polyethylene (HDPE). Alternatively, it may also be a composite material consisting of a metal, a polymer and an inorganic non-metallic material.

Fig. 26 is a schematic diagram showing the structure of the helical layer and the braided layer, which form an angle therebetween, according to an embodiment of the present application. As shown in Fig. 26, the first filaments form an angle a with the second filaments. Specifically, the angle a between the first and second filaments in at least one segment of the delivery sheath 400 may differ from that in another segment. In this way, the distal segment of the delivery sheath 400 has desirable compliance, tensile strength and radial strength, and the intermediate segment thereof can avoid the distal and proximal segments from experiencing stress jumps. Moreover, the proximal segment of the delivery sheath 400 exhibits high strength and good pushability, ensuring that the intravascular interventional device can be released and retrieved. The angle a between the first and second filaments in the distal segment may be smaller than that in the proximal segment. The angle a between the first and second filaments may range from 10° to 50°. Preferably, the angle a between the first and second filaments ranges from 15° to 25° in the distal segment. The angle a between the first and second filaments may range from 35° to 45° in the proximal segment, which imparts enhanced resistance to axial forces and reduced axial elongation to the delivery sheath 400. It is to be noted that the angle is defined as an angle between axial projections of the first and the second filaments.

Optionally, the embolic protection device may further include a plurality of radiographically visible elements, which make it possible to clearly discern whether the embolic diverter 100 is being expanded or collapsed during the surgical procedure. The radiographically visible elements may be dots or wires. The radiographically visible elements are disposed, for example, on the embolic diverter 100. Specifically, the radiographically visible elements may be disposed on both ends of the first support frame 110, optionally in a continuous pattern. Additionally, the radiographically visible elements may be also provided on the delivery sheath 400, in particular, at the distal end thereof. The radiographically visible elements may be also provided on the push tube 300, in particular, at the distal end thereof. The radiographically visible elements can help the operator to confirm the position of the embolic protection device during its delivery, placement, release or the like in the course of the surgical procedure. The radiographically visible element may be formed of tantalum, platinum, gold, tungsten or another material.

Optionally, the embolic protection device further includes a guide member 500 coupled to the proximal end of the embolic diverter 100. The guide member 500 defines, at its proximal end, an inwardly tapered structure for assisting in anchoring of the embolic diverter 100 in the aortic arch without displacement and for preventing the guide member 500 from causing damage to the blood vessel wall.

The present application also provides another embolic protection system. Referring to Figs. 23 and 24, the embolic protection system includes a push tube 300, an embolic diverter 100 and an embolic collector 200. The push tube 300 has a first proximal end and an opposing first distal end. The embolic diverter 100 is provided on the push tube 300. The embolic collector 200 is coaxially disposed on the push tube 300 between the first proximal end and the embolic diverter 100.

The embolic protection system further includes an outer sheath 400 defining a first lumen extending axially therethrough. The first lumen is adapted to accommodate the push tube 300, as well as the embolic diverter 100 and the embolic collector 200, both in a crimped configuration.

Optionally, the embolic protection system may further include a handle 700 for facilitating a physician's operation for delivery and retrieval. Both the push tube 300 and the outer sheath 400 may be coupled to the handle 700 as is conventional. The handle 700 is also provided with a contrast catheter inlet (not shown).

In summary, the present application provides an embolic protection device including: an embolic diverter and a push tube, the embolic diverter including a first support frame and a first filter member provided on the first support frame; and a push tube including a tubular body coupled to the embolic diverter, the push tube adapted to advance, position and adjust the embolic diverter, the tubular body having a distal cut hypotube section. According to the present application, the push tube enables the embolic diverter that is intended to protect upstream blood vessels to have excellent wall conformity. The distal cut hypotube section of the tubular body allows the push tube to be shaped by a guidewire disposed therein and thus self-adapt to the anatomy of a patient's aortic arch and additionally enhance the embolic diverter's wall conformity. The push tube further includes a first coupling element defining a through hole, through which the first support frame or the first filter member is proximally passed, thus coupling the embolic diverter to the push tube. The first coupling element enables the push tube to deliver better pushing and manipulating forces, imparting desirable degrees of freedom to the embolic protection device. The embolic protection device is simple in design, comprehensive in functionality and convenient to manipulate.

It is to be noted that, as used herein, the terms "first", "second", "third", "fourth" and the like are only meant to distinguish various components, elements, steps, etc. from each other rather than indicate logical or sequential orderings thereof, unless otherwise indicated or specified.

It is to be understood that while the invention has been described above with reference to preferred embodiments thereof, it is not limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. An embolic protection device, comprising:
an embolic diverter comprising a first support frame and a first filter member disposed on the first support frame; and
a push tube comprising a tubular body connected to the embolic diverter, the tubular body configured to advance, position and adjust the embolic diverter.

2. The embolic protection device of claim 1, wherein the push tube further comprises a first coupling element provided on the tubular body, and wherein the tubular body is connected to the embolic diverter by the first coupling element.

3. The embolic protection device of claim 1, wherein the first coupling element defines a through hole, through which a proximal end of the first support frame or the first filter member is passed, thereby connecting the embolic diverter to the push tube.

4. The embolic protection device of claim 3, wherein the through hole extends through the tubular body in a direction perpendicular to an axis of the tubular body.

5. The embolic protection device of claim 3, wherein the first coupling element comprises a first tubular element, a first connecting bar and a second tubular element, which are sequentially arranged along a direction from a distal end of the tubular body to a proximal end thereof, the first and second tubular elements both coaxially disposed with the tubular body and sleeved on the tubular body, and the first and second tubular elements both connected to each other by the first connecting bar.

6. The embolic protection device of claim 5, wherein the first connecting bar is elongate and convex away from the tubular body, thus forming the through hole between the first connecting bar and the tubular body.

7. The embolic protection device of claim 3, wherein the first coupling element comprises a third tubular element, a second connecting bar and a fourth tubular element, which are sequentially arranged along a direction from a distal end of the tubular body to a proximal end thereof, the fourth tubular element disposed coaxially with the tubular body and sleeved on the tubular body, the third tubular element connected to the fourth tubular element by the second connecting bar, the third tubular element having an axis that is perpendicular to an axis of the tubular body, the third tubular element defining a lumen which provides the through hole.

8. The embolic protection device of claim 1, wherein the tubular body further comprises a cut hypotube section disposed at a distal end thereof, the cut hypotube section having a length between 80 mm and 350 mm along an axis of the tubular body.

9. The embolic protection device of claim 8, wherein the cut hypotube section comprises a plurality of cut units extending helically along the axis of the tubular body, or wherein the cut hypotube section comprises a plurality of cut portions spaced apart along the axis of the tubular body, each comprising at least one cut unit spaced apart circumferentially around the tubular body, or wherein the cut hypotube section comprises a plurality of cut portions spaced apart circumferentially around the tubular body, each comprising at least one cut unit spaced apart along the axis of the tubular body.

10. The embolic protection device of claim 9, wherein each cut unit comprises at least one groove, the at least one groove comprising at least one circular recess at an end thereof and an elongate groove connecting the circular recess, the elongate groove comprising at least one V-shaped structure and brought into communication with the circular recess, or wherein each cut unit comprises at least two grooves spaced apart along the direction of extension of the specific cut unit.

11. The embolic protection device of claim 10, wherein the direction of extension of each cut unit forms an angle ranging from 60 degrees to 90 degrees with the axis of the tubular body.

12. The embolic protection device of claim 10, wherein a ratio of a length of each cut unit extending on an outer surface of the tubular body to a circumferential length of the outer surface of the tubular body ranges from 0.08 to 0.90.

13. The embolic protection device of claim 1, wherein the embolic protection device further comprises an embolic collector comprising a second support frame and a second filter member attached to the second support frame, wherein the push tube further comprises a second coupling element disposed on the tubular body between the first coupling element and a proximal end of the tubular body, and wherein the embolic collector is coupled to the push tube by the second coupling element.

14. The embolic protection device of claim 13, wherein the second coupling element comprises a groove running circumferentially around the tubular body, and wherein the embolic collector is disposed at the groove and thus connected to the push tube.

15. The embolic protection device of claim 1, wherein the embolic protection device further comprises an embolic collector comprising:
a proximal connection;
a second support frame comprising a plurality of support struts spaced apart about an axis, at least some of which are curved to define a concave surface facing the axis, each of the support struts having a first proximal end and a first distal end opposing the first proximal end along a direction parallel to the axis, the first proximal ends of the support struts gathered at the proximal connection; and
a second filter member having a second proximal end and a second distal end opposing the second proximal end, the second filter member provided on the second support frame, the second proximal end provided at the first proximal ends of the support struts, the second distal end provided on the second support frame to form an opening.

16. The embolic protection device of claim 1, wherein the push tube is a straight structure having an outer diameter kept constant from a proximal end of the push tube to a distal end thereof, or wherein the push tube is a tapered structure that it is wide at the proximal end and narrow at the distal end.

17. The embolic protection device of claim 1, wherein the embolic diverter further comprises at least one first support strut connected to the first support frame, and connection points of the first support strut and the first support frame scattered on opposite sides of a longitudinal centerline of the first support frame.

18. The embolic protection device of claim 1, wherein the first filter member has at least two concave surfaces all facing a plane defined by the support frame.

19. The embolic protection device of claim 1, further comprising a delivery sheath comprising an inner layer, and both disposed externally around the inner layer, a helical layer and a braided layer, the braided layer being a meshed structure braided from interlaced first filaments, the helical layer being a helical structure formed by axially coiled second filaments, the delivery sheath comprising at least two axially-extending segments, in at least one of which, an angle formed by the first and second filaments differs from an angle formed by the first and second filaments in another one of the segments.

20. An embolic protection device, comprising:
a push tube having a first proximal end and a first distal end opposing the first proximal end;
an embolic diverter provided on the push tube, the embolic diverter comprising a first filter member; and
an embolic collector coaxially disposed on the push tube between the embolic diverter and the first proximal end, the embolic collector comprising a second support member and a second filter member provided on the second support member, the second filter member having a second proximal end and a second distal end opposing the second proximal end, the second filter member being closed at the first proximal end and defining an opening at the second distal end, the opening configured to allow clots to enter and thus be trapped in the second filter member.
